# EUROPÄISCHE PATENTSCHRIFT

(11) **EP 1 237 881 B1**
(45) Veröffentlichungstag und Bekanntmachung des Hinweises auf die Patenterteilung: **03.11.2004**
(21) Anmeldenummer: 00977583.4
(22) Anmeldetag: 27.11.2000
(51) Int. Cl.: C07D 261/04, A01N 43/80

(54) **HERBIZIDE HALOGENALKYL-SUBSTITUIERTE 3-(4,5-DIHYDROISOXAZOL-3-YL)-BENZOYL-CYCLOHEXENONE**
HERBICIDAL HALOGENALKYL-SUBSTITUTED 3-(4,5-DIHYDROISOXAZOL-3-YL)-BENZOYL-CYCLOHEXENONES
3-(4,5-DIHYDROISOXAZOL-3-YL)-BENZOYL-CYCLOHEXENONES SUBSTITUEES PAR UN HALOGENALKYLE ET A ACTION HERBICIDE

(30) Priorität: 02.12.1999 DE 19958034
(43) Veröffentlichungstag der Anmeldung: 11.09.2002
(73) Patentinhaber: BASF AKTIENGESELLSCHAFT, 67056 Ludwigshafen (DE)
(72) Erfinder: KUDIS, Steffen, 68167 Mannheim (DE); BAUMANN, Ernst, 67373 Dudenhofen (DE); VON DEYN, Wolfgang, 67435 Neustadt (DE); LANGEMANN, Klaus, 34270 Schauenburg (DE); MAYER, Guido, 67161 Gönnheim (DE); MISSLITZ, Ulf, 67433 Neustadt (DE); NEIDLEIN, Ulf, 68165 Mannheim (DE); WITSCHEL, Matthias, 67098 Bad Dürkheim (DE); WESTPHALEN, Karl-Otto, 67346 Speyer (DE); WALTER, Helmut, 67283 Obrigheim (DE)
(86) Internationale Anmeldenummer: PCT/EP2000/011818
(87) Internationale Veröffentlichungsnummer: WO 2001/040199

(56) Entgegenhaltungen:
- WO-A-96/26200

## Beschreibung

Die vorliegende Erfindung betrifft Halogenalkyl-substituierte 3-(4, 5-Dihydroisoxazol-3-yl)-Benzoylcyclohexenone sowie Verfahren zu ihrer Herstellung, Mittel, welche diese enthalten, sowie die Verwendung dieser Derivate oder Mittel zur Schadpflanzenbekämpfung.

Aus der WO 96/26200 sind herbizide 2-Benzoylcyclohexan-1,3-dione bekannt.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden Halogenalkyl-substituierte 3-(4,5-Dihydroisoxazol-3-yl)-Benzoylcyclohexenone der Formel I 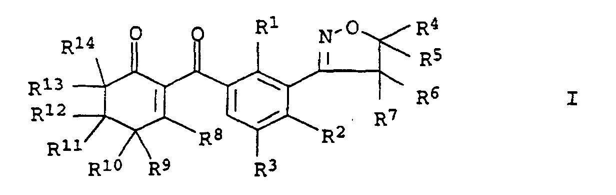 worin
- R¹: für C₁-C₆-Alkyl, C₁-C₆-Halogenalkyl, C₁-C₆-Alkoxy oder C₁-C₆-Halogenalkoxy;
- R²: für C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl, Halogen, Cyano oder Nitro;
- R³: für Wasserstoff, C₁-C₆-Alkyl oder Halogen;
- R⁴: für C₁-C₄-Halogenalkyl steht;
- R⁵, R⁶, R⁷: unabhängig voneinander für Wasserstoff, C₁-C₄-Alkyl oder C₁-C₄-Halogenalkyl stehen;
- R⁸: für Hydroxy, Mercapto, Halogen, OR¹⁵, SR¹⁵, SOR¹⁶ oder SO₂R¹⁶ steht;
- R⁹, R¹³: unabhängig voneinander für Wasserstoff, C₁-C₄-Alkyl, C₁-C₄-Alkylthio oder C₁-C₄-Alkoxycarbonyl stehen;
- R¹⁰, R¹², R¹⁴: unabhängig voneinander für Wasserstoff oder C₁-C₄-Alkyl stehen;
- R¹¹: für Wasserstoff, Hydroxy, Halogen, C₁-C₆-Alkyl, C₁-C₆-Halogenalkyl, Di(C₁-C₆-alkoxy)methyl, (C₁-C₆-Alkoxy) (C₁-C₆-alkylthio)methyl, Di(C₁-C₆-alkylthio)methyl, C₁-C₆-Alkoxy, C₁-C₆-Halogenalkoxy, C₁-C₆-Alkylthio, C₁-C₆-Halogenalkylthio, C₁-C₆-Alkoxycarbonyl, C₁-C₆-Halogenalkoxycarbonyl, 1,3-Dioxolan-2-yl, 1,3-Dioxan-2-yl, 1,3-Oxathiolan-2-yl, 1,3-Oxathian-2-yl, 1,3-Dithiolan-2-yl oder 1,3-Dithian-2-yl steht, wobei die sechs letztgenannten Reste einen, zwei oder drei unter C₁-C₄-Alkyl ausgewählte Substituenten tragen könnnen;
oder
- R⁹ und R¹⁰ oder R¹³ und R¹⁴: gemeinsam für C₁-C₅-Alkandiyl stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
- R¹⁰ und R¹¹ oder R¹¹ und R¹⁴: gemeinsam für eine chemische Bindung oder C₁-C₅-Alkandiyl stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
oder
- R¹⁰ und R¹⁴: gemeinsam für C₁-C₄-Alkandiyl stehen, das einen, zwei oder drei unter Halogen, Cyano, C₁-C₄-Alkyl, C₁-C₄-Alkoxycarbonyl ausgewählte Substituenten tragen kann;
oder
- R¹¹ und R¹²: gemeinsam für -O-(CH₂)ₚ-O-, -O-(CH₂)ₚ-S-, -S-(CH₂)ₚ-S-, -O-(CH₂)_{q}- oder -S-(CH₂)_{q}- stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
oder
- R¹¹ und R¹²: gemeinsam für ein Sauerstoffatom stehen;
- R¹⁵: für C₁-C₆-Alkyl, C₃-C₆-Alkenyl, C₃-C₆-Halogenalkenyl, C₃-C₆-Alkinyl, C₃-C₆-Cycloalkyl, C₁-C₂₀-Alkylcarbonyl, C₂-C₂₀-Alkenylcarbonyl, C₂-C₆-Alkinylcarbonyl, C₁-C₆-Alkoxycarbonyl, C₃-C₆-Alkenyloxycarbonyl, C₃-C₆-Alkinyloxycarbonyl, C₁-C₆-Alkylthiocarbonyl, C₁-C₆-Alkylaminocarbonyl, C₃-C₆-Alkenylaminocarbonyl, C₃-C₆-Alkinylaminocarbonyl, N,N-Di(C₁-C₆-alkyl)aminocarbonyl, N-(C₃-C₆-Alkenyl)-N-(C₁-C₆-alkyl) aminocarbonyl, N-(C₃-C₆-Alkinyl)-N-(C₁-C₆-alkyl)aminocarbonyl, N-(C₁-C₆-Alkoxy)-N-(C₁-C₆-alkyl)aminocarbonyl, N,N-Di(C₁-C₆-alkyl)aminothiocarbonyl, C₁-C₆-Alkoxyimino-C₁-C₆-alkyl steht, wobei die genannten Alkyl-, Alkoxy- und Cycloalkylreste partiell oder vollständig halogeniert sein können und/oder einen, zwei oder drei unter Cyano, C₁-C₄-Alkoxy, C₁-C₄-Alkylthio, Di(C₁-C₄-alkyl)amino, C₁-C₄-Alkylcarbonyl, C₁-C₄-Alkoxycarbonyl, C₁-C₄-Alkoxy-C₁-C₄-alkoxycarbonyl, C₁-C₄-Alkylaminocarbonyl, N,N-Di-(C₁-C₄-alkyl)aminocarbonyl oder C₃-C₆-Cycloalkyl ausgewählte Substituenten tragen können; Phenyl, Phenyl-C₁-C₆-alkyl, Phenylcarbonyl-C₁-C₆-alkyl, Phenylcarbonyl, Phenoxycarbonyl, Phenoxythiocarbonyl, Heterocycyl, Heterocyclyl-C₁-C₆-alkyl, Heterocyclylcarbonyl-C₁-C₆-alkyl, Heterocyclylcarbonyl, Heterocyclyloxycarbonyl, Heterocyclyloxythiocarbonyl, wobei der Phenyl- oder Heterocyclylrest der genannten Reste partiell oder vollständig halogeniert sein kann und/oder einen, zwei oder drei unter Nitro, Cyano, C₁-C₄-Alkyl, C₁-C₄-Halogenalkyl, C₁-C₄-Alkoxy oder C₁-C₄-Halogenalkoxy ausgewählte Substituenten tragen kann;
- R¹⁶: für C₁-C₆-Alkyl, C₃-C₆-Alkenyl, C₃-C₆-Halogenalkenyl, C₃-C₆-Alkinyl oder C₃-C₆-Cycloalkyl steht, wobei die genannten Alkyl- und Cycloalkylreste partiell oder vollständig halogeniert sein können und/oder einen, zwei oder drei unter Cyano, C₁-C₄-Alkoxy, C₁-C₄-Alkylthio, Di(C₁-C₄-alkyl)amino, C₁-C₄-Alkylcarbonyl, C₁-C₄-Alkoxycarbonyl, C₁-C₄-Alkoxy-C₁-C₄-alkoxycarbonyl, C₁-C₄-Alkylaminocarbonyl, N,N-Di-(C₁-C₄-alkyl)aminocarbonyl oder C₃-C₆-Cycloalkyl ausgewählte Substituenten tragen können;
Phenyl, Phenyl-C₁-C₆-alkyl, Phenylcarbonyl-C₁-C₆-alkyl, Heterocycyl, Heterocyclyl-C₁-C₆-alkyl oder Heterocyclylcarbonyl-C₁-C₆-alkyl, wobei der Phenyl- oder Heterocyclylrest der genannten Reste partiell oder vollständig halogeniert sein kann und/oder einen, zwei oder drei unter Nitro, Cyano, C₁-C₄-Alkyl, C₁-C₄-Halogenalkyl, C₁-C₄-Alkoxy oder C₁-C₄-Halogenalkoxy ausgewählte Substituenten tragen kann;
- p: für 2, 3 oder 4;
- q: für 1, 2, 3, 4 oder 5 steht;
sowie deren landwirtschaftlich brauchbaren Salze gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische. Die Verbindungen der Formel I mit R⁸ = OH oder SH können auch als Tautomere zu der dargestellten Struktur oder als Tautomerengemische vorliegen.

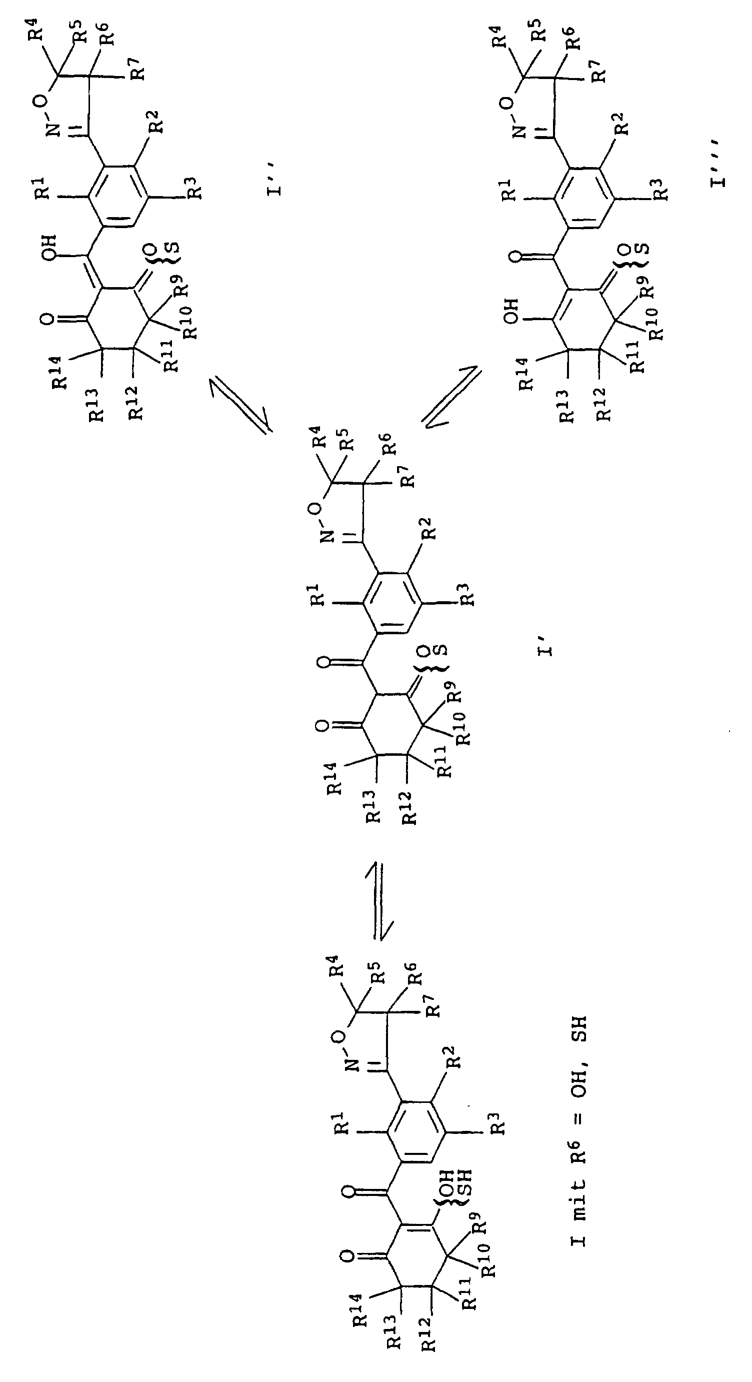

Die Verbindungen der Formel I können auch in Form ihrer landwirtschaftlich brauchbaren Salze vorliegen, wobei es auf die Art des Salzes in der Regel nicht ankommt. Im Allgemeinen kommen die salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R¹⁶ oder als Reste an Phenylringen genannten organischen Molekülteile stellen Sammelbegriffe für individuelle Aufzählungen der einzelnen Gruppenmitglieder dar. Sämtliche Kohlenwasserstoffketten, also Alkyl-, Halogenalkyl-, Dialkoxymethyl-, Alkoxyalkylthiomethyl-, Dialkylthiomethyl-, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, Dialkylamino-, Alkylcarbonyl-, Alkoxycarbonyl-, Halogenalkoxycarbonyl-, Alkylthiocarbonyl-, Alkoxyalkoxycarbonyl-, Alkylaminocarbonyl-, Dialkylaminocarbonyl-, Dialkylaminothiocarbonyl, Alkoxyiminoalkyl, N-Alkoxy-N-alkylaminocarbonyl-, Phenylalkyl-, Heterocyclylalkyl-, Phenylcarbonylalkyl-, Heterocyclylcarbonylalkyl-, Alkenylcarbonyl-, Alkenyloxycarbonyl-, Alkenylaminocarbonyl-, N-Alkenyl-N-alkylaminocarbonyl-, Alkinylcarbonyl-, Alkinyloxycarbonyl-, Alkinylaminocarbonyl-, N-Alkinyl-N-alkylaminocarbonyl, Alkenyl-, Alinyl-, Halogenalkenyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl, sowie die Alkylteile von C₁-C₄-Alkylcarbonyl, Phenyl-C₁-C₄-alkyl-, Phenylcarbonyl-C₁-C₄-alkyl, Heterocyclyl-C₁-C₄-alkyl und Heterocyclylcarbonyl-C₁-C₄-alkyl: z. B. Methyl, Ethyl, Propyl, 1-Methylethyl, Butyl, 1-Methylpropyl, 2-Methylpropyl und 1,1-Dimethylethyl;
- C₁-C₆-Alkyl, sowie die Alkylteile von C₁-C₆-Alkylcarbonyl, N-C₃-C₆-Alkenyl-N-C₁-C₆-alkylaminocarbonyl-, N-C₃-C₆-Alkinyl-N-alkylaminocarbonyl-, N-C₁-C₆-Alkoxy-N-C₁-C₆-alkylaminocarbonyl-, Phenyl-C₁-C₆-alkyl-, Phenylcarbonyl-C₁-C₆-alkyl, Heterocyclyl-C₁-C₆-alkyl, Heterocyclylcarbonyl-C₁-C₆-alkyl und C₁-C₆-Alkoxyimino-C₁-C₆-alkyl: C₁-C₄-Alkyl, wie voranstehend genannt, sowie z. B. Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₂₀-Alkyl als Alkylteil von C₁-C₂₀-Alkylcarbonyl: C₁-C₆-Alkyl, wie voranstehend genannt, sowie Heptyl, Octyl, Pentadecyl oder Heptadecyl;
- C₁-C₂-Halogenalkyl: einen Methyl- oder Ethylrest, wie voranstehend genannt, der partiell oder vollständig durch Fluor, Chlor, Brom und/oder Iod substituiert ist, also z. B. Chlormethyl, Brommethyl, Iodmethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl oder Pentafluorethyl;
- C₁-C₄-Halogenalkyl: C₁-C₂-Halogenalkyl, wie voranstehend genannt, sowie z. B. 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl: C₁-C₄-Halogenalkyl, wie voranstehend genannt, sowie z. B. 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl oder Dodecafluorhexyl;
- C₁-C₄-Alkoxy, sowie die Alkoxyteile von C₁-C₄-Alkoxycarbonyl und C₁-C₄-Alkoxy-C₁-C₄-alkoxycarbonyl: z. B. Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy, sowie die Alkoxyteile von C₁-C₆-Alkoxycarbonyl, Di(C₁-C₆-Alkoxy)methyl, (C₁-C₆-Alkoxy) (C₁-C₆-alkylthio) methyl, N-(C₁-C₆-Alkoxy)-N-(C₁-C₆-alkyl)aminocarbonyl und C₁-C₆-Alkoxyimino-C₁-C₆-alkyl: C₁-C₄-Alkoxy, wie voranstehend genannt, sowie z. B. Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy,1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₄-Halogenalkoxy: einen C₁-C₄-Alkoxyrest, wie voranstehend genannt, der partiell oder vollständig durch Fluor, Chlor, Brom und/oder Iod substituiert ist, also z. B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₆-Halogenalkoxy, sowie die Halogenalkoxyteile von C₁-C₆-Halogenälkoxycarbonyl: C₁-C₄-Halogenalkoxy, wie voranstehend genannt, sowie z. B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy oder Dodecafluorhexoxy;
- C₁-C₄-Alkylthio: z. B. Methylthio, Ethylthio, Propylthio, 1-Methylethylthio, Butylthio, 1-Methylpropylthio, 2-Methylpropylthio und 1,1-Dimethylethylthio;
- C₁-C₆-Alkylthio, sowie die Alkylthioteile von (C₁-C₆-Alkoxy) (C₁-C₆-alkylthio)methyl, Di(C₁-C₆-alkylthio)methyl und (C₁-C₆-Alkylthio)carbonyl: C₁-C₄-Alkylthio, wie voranstehend genannt, sowie z. B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio oder 1-Ethyl-2-methylpropylthio;
- C₁-C₄-Halogenalkylthio: einen C₁-C₄-Alkylthiorest, wie voranstehend genannt, der partiell oder vollständig durch Fluor, Chlor, Brom und/oder Iod substituiert ist, also z. B. Fluormethylthio, Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio und Nonafluorbutylthio;
- C₁-C₆-Halogenalkylthio: C₁-C₄-Halogenalkylthio, wie voranstehend genannt, sowie z. B. 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio oder Dodecafluorhexylthio;
- C₁-C₆-Alkylsulfinyl (C₁-C₆-Alkyl-S(=O)-): z. B. Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl oder 1-Ethyl-2-methylpropylsulfinyl;
- C₁-C₆-Halogenalkylsulfinyl: C₁-C₆-Alkylsulfinylrest, wie voranstehend genannt, der partiell oder vollständig durch Fluor, Chlor, Brom und/oder Iod substituiert ist, also z. B. Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl, Nonafluorbutylsulfinyl, 5-Fluorpentylsulfinyl, 5-Chlorpentylsulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl oder Dodecafluorhexylsulfinyl;
- C₁-C₄-Alkylsulfonyl (C₁-C₄-Alkyl-S(=O)₂-), sowie die Alkylsulfonylteile von C₁-C₄-Alkylsulfonyloxy: z. B. Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
- C₁-C₆-Alkylsulfonyl: einen C₁-C₄-Alkylsulfonylrest, wie voranstehend genannt, sowie z. B. Pentylsulfonyl, 2-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
- C₁-C₆-Halogenalkylsulfonyl: einen C₁-C₆-Alkylsulfonylrest, wie voranstehend genannt, der partiell oder vollständig durch Fluor, Chlor, Brom und/oder Iod substituiert ist, also z. B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl oder Dodecafluorhexylsulfonyl;
- Di-(C₁-C₄-alkyl)amino, sowie die Dialkylaminoteile von Di(C₁-C₄-alkyl)aminocarbonyl: N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl) amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)-amino, N-Butyl-N-(2-methylpropyl)amino, N-Butyl-N-(1,1-dimethylethyl)amino, N-(1-Methylpropyl)-N-(2-methylpropyl)-amino, N-(1,1-Dimethylethyl)-N-(1-methylpropyl)-amino oder N-(1,1-Dimethylethyl)-N-(2-methylpropyl)amino;
- Di(C₁-C₆-alkylamino) als Dialkylaminoteil von Di(C₁-C₆-alkyl)aminocarbonyl und Di(C₁-C₆-alkyl)aminothiocarbonyl: N-Methyl-N-pentylamino oder N-Methyl-N-hexylamino;
- (C₁-C₄-Alkylamino)carbonyl: z. B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- (C₁-C₆-Alkylamino)carbonyl: (C₁-C₄-Alkylamino)carbonyl, wie vorstehend genannt, sowie z. B. Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl oder 1-Ethyl-2-methylpropylaminocarbonyl;
- C₃-C₆-Alkenyl, sowie die Alkenylteile von C₃-C₆-Alkenylcarbonyl, C₃-C₆-Alkenyloxycarbonyl, C₃-C₆-Alkenylaminocarbonyl, N-(C₃-C₆-Alkenyl)-N-(C₁-C₆-alkyl)aminocarbonyl :
   Prop-1-en-1-yl, Prop-2-en-1-yl, 1-Methylethenyl, Buten-1-yl, Buten-2-yl, Buten-3-yl, 1-Methyl-prop-1-en-1-yl, 2-Methylprop-1-en-1-yl, 1-Methyl-prop-2-en- 1-yl, 2-Methylprop-2-en-1-yl, Penten-1-yl, Penten-2-yl, Penten-3-yl, Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methylbut-2-en-1-yl, 3-Methyl-but-2-en-1-yl, 1-Methylbut-3-en-1-yl, 2-Methyl-but-3-en-1-yl, 3-Methylbut-3-en-1-yl, 1,1-Dimethyl-prop-2-en-1-yl, 1,2-Dimethylprop-1-en-1-yl, 1,2-Dimethyl-prop-2-en-1-yl, 1-Ethylprop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethylbut-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methylprop-1-en-1-yl und 1-Ethyl-2-methyl-prop-2-en-1-yl;
- C₂-C₆-Alkenyl als Alkenylteile von C₂-C₆-Alkenylcarbonyl: C₃-C₆-Alkenyl, wie voranstehend genannt, sowie Ethenyl;
- C₂-C₂₀-Alkenyl als Alkenylteile von C₂-C₂₀-Alkenylcarbonyl: C₂-C₆-Alkenyl, wie voranstehend genannt, sowie 8-Pentadecen-1-yl, 8-Heptadecen-1-yl und 8,11-Heptadecadien-1-yl;
- C₃-C₆-Halogenalkenyl: einen C₃-C₆-Alkenylrest, wie voranstehend genannt, der partiell oder vollständig durch Fluor, Chlor, Brom und/oder Iod substituiert ist, also z. B. 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl;
- C₃-C₆-Alkinyl, sowie die Alkinylteile von C₃-C₆-Alkinylcarbonyl, C₃-C₆-Alkinyloxycarbony, C₃-C₆-Alkinylaminocarbonyl, N-(C₃-C₆-Alkinyl)-N-(C₁-C₆-alkyl)-aminocarbonyl: z. B. Propargyl, But-1-in-3-yl, But-1-in-4-yl, But-2-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methylbut-1-in-3-yl, 3-Methyl-but-1-in-4-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-3-yl, 3-Methylpent-1-in-4-yl, 3-Methyl-pent-1-in-5-yl, 4-Methylpent-2-in-4-yl oder 4-Methyl-pent-2-in-5-yl;
- C₂-C₆-Alkinyl als Alkinylteil von C₂-C₆-Alkinylcarbonyl: C₃-C₆-Alkinyl, wie voranstehend genannt, sowie Ethinyl;
- C₁-C₄-Alkandiyl: z. B. Methandiyl, Ethan-1,2-diyl, Propan-1,3-diyl oder Butan-1,4-diyl;
- C₁-C₅-Alkandiyl: C₁-C₄-Alkandiyl, wie vorstehend genannt, sowie Pentan-1,5-diyl;
- C₃-C₆-Cycloalkyl, sowie die Cycloalkylteile von C₃-C₆-Cycloalkylcarbonyl: z. B. Cyclopropyl, Cyclobutyl, Cyclopentyl oder Cyclohexyl;
- Heterocyclyl, sowie Heterocyclylteile von Heterocyclylcarbonyl, Heterocyclyl-C₁-C₆-alkyl, Heterocyclyloxycarbonyl, Heterocyclyloxythiocarbonyl, Heterocyclylcarbonyl-C₁-C₆-alkyl: ein gesättigter, partiell gesättigter oder ungesättigter 5- oder 6-gliedriger heterocyclischer Ring, der ein bis vier gleiche oder verschiedene Heteroatome, ausgewählt aus folgender Gruppe: Sauerstoff, Schwefel oder Stickstoff, enthält, und über C oder N gebunden sein kann, also z. B. C-gebundene 5-gliedrige, gesättigte Ringe, wie:
   Tetrahydrofuran-2-yl, Tetrahydrofuran-3-yl, Tetrahydrothien-2-yl, Tetrahydrothien-3-yl,Tetrahydropyrrol-2-yl, Tetrahydropyrrol-3-yl, Tetrahydropyrazol-3-yl, Tetrahydropyrazol-4-yl, Tetrahydroisoxazol-3-yl, Tetrahydroisoxazol-4-yl, Tetrahydroisoxazol-5-yl, 1,2-Oxathiolan-3-yl, 2,2-Oxathiolan-4-yl, 1,2-Oxathiolan-5-yl, Tetrahydroisothiazel-3-yl, Tetrahydroisothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4-yl, Tetrahydrooxazol-2-yl, Tetrahydrooxazol-4-yl, Tetrahydrooxazol-5-yl, Tetrahydrothiazol-2-yl, Tetrahydrothiazol-4-yl, Tetrahydrothiazol-5-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl, 1,3,2-Dioxathiolan-4-yl;
   C-gebundene, 5-gliedrige, partiell gesättigte Ringe, wie:
      2,3-Dihydrofuran-2-yl, 2,3-Dihydrofuran-3-yl, 2,5-Dihydrofuran-2-yl, 2,5-Dihydrofuran-3-yl, 4,5-Dihydrofuran-2-yl, 4,5-Dihydrofuran-3-yl, 2,3-Dihydrothien-2-yl, 2,3-Dihydrothien-3-yl, 2,5-Dihydrothien-2-yl, 2,5-Dihydrothien-3-yl, 4,5-Dihydrothien-2-yl, 4,5-Dihydrothien-3-yl, 2,3-Dihydro-1H-pyrrol-2-yl, 2,3-Dihydro-1H-pyrrol-3-yl, 2,5-Dihydro-1H-pyrrol-2-yl, 2,5-Dihydro-1Hpyrrol-3-yl, 4,5-Dihydro-1H-pyrrol-2-yl, 4,5-Dihydro-1Hpyrrol-3-yl, 3,4-Dihydro-2H-pyrrol-2-yl, 3,4-Dihydro-2Hpyrrol-3-yl, 3,4-Dihydro-5H-pyrrol-2-yl, 3,4-Dihydro-5Hpyrrol-3-yl, 4,5-Dihydro-1H-pyrazol-3-yl, 4,5-Dihydro-1Hpyrazol-4-yl, 4,5-Dihydro-1H-pyrazol-5-yl, 2,5-Dihydro-1Hpyrazol-3-yl, 2,5-Dihydro-1H-pyrazol-4-yl, 2,5-Dihydro-1Hpyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ³-1,2-Dithiol-3-yl, Δ³-1,2-Dithiol-4-yl, Δ³-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrooxazol-2-yl, 2,3-Dihydrooxazol-4-yl, 2,3-Dihydrooxazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydrothiazol-2-yl, 2,3-Dihydrothiazol-4-yl, 2,3-Dihydrothiazol-5-yl, 1,3-Dioxol-2-yl, 1,3-Dioxol-4-yl, 1,3-Dithiol-2-yl, 1,3-Dithiol-4-yl, 1,3-Oxathiol-2-yl, 1,3-Oxathiol-4-yl, 1,3-Oxathiol-5-yl, 1,2,3-Δ²-Oxadiazolin-4-yl, 1,2,3-Δ²-Oxadiazolin-5-yl, 1,2,4-Δ⁴-Oxadiazolin-3-yl, 1,2,4-Δ⁴-Oxadiazolin-5-yl, 1,2,4-Δ²-Oxadiazolin-3-yl, 1,2,4-Δ²-Oxadiazolin-5-yl, 1,2,4-Δ³-Oxadiazolin-3-yl, 1,2,4-Δ³-Oxadiazolin-5-yl, 1,3,4-Δ²-Oxadiazolin-2-yl, 1,3,4-Δ²-Oxadiazolin-5-yl, 1,3,4-Δ³-Oxadiazolin-2-yl, 1,3,4-Oxadiazolin-2-yl, 1,2,4-Δ⁴-Thiadiazolin-3-yl, 1,2,4-Δ⁴-Thiadiazolin-5-yl, 1,2,4-Δ³-Thiadiazolin-3-yl, 1,2,4-Δ³-Thiadiazolin-5-yl, 1,2,4-Δ²-Thiadiazolin-3-yl, 1,2,4-Δ²-Thiadiazolin-5-yl, 1,3,4-Δ²-Thiadiazolin-2-yl, 1,3,4-Δ²-Thiadiazolin-5-yl, 1,3,4-Δ³-Thiadiazolin-2-yl, 1,3,4-Thiadiazolin-2-yl, 1,2,3-Δ²-Triazolin-4-yl, 1,2,3-Δ²-Triazolin-5-yl, 1,2,4-Δ²-Triazolin-3-yl, 1,2,4-Δ²-Triazolin-5-yl, 1,2,4-Δ³-Triazolin-3-yl, 1,2,4-Δ³-Triazolin-5-yl, 1,2,4-Δ¹-Triazolin-2-yl, 1,2,4-Triazolin-3-yl, 3H-1,2,4-Dithiazol-5-yl, 2H-1,3,4-Dithiazol-5-yl, 2H-1,3,4-Oxathiazol-5-yl;
   C-gebundene, 5-gliedrige, ungesättigte Ringe, wie:
      2-Furyl, 3-Furyl, 2-Thienyl, 3-Thienyl, Pyrrol-2-yl, Pyrrol-3-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl, Thiazol-5-yl, 1,2,3-Oxadiazol-4-yl, 1,2,3-Oxadiazol-5-yl, 1,2,4-Oxadiazol-3-yl, 1,2,4,-Oxadiazol-5-yl, 1,3,4-Oxadiazol-2-yl, 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl, 1,2,3-Triazol-4-yl, 1,2,4-Triazol-3-yl, Tetrazol-5-yl;
   C-gebundene, 6-gliedrige, gesättigte Ringe, wie:
      Tetrahydropyran-2-yl, Tetrahydropyran-3-yl, Tetrahydropyran-4-yl, Piperidin-2-yl, Piperidin-3-yl, Piperidin-4-yl, Tetrahydrothiopyran-2-yl, Tetrahydrothiopyran-3-yl, Tetrahydrothiopyran-4-yl, 1,3-Dioxan-2-yl, 1,3-Dioxan-4-yl, 1,3-Dioxan-5-yl, 1,4-Dioxan-2-yl, 1,3-Dithian-2-yl, 1,3-Dithian-4-yl, 1,3-Dithian-5-yl, 1,4-Dithian-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, 1,2-Dithian-3-yl, 1,2-Dithian-4-yl, Hexahydropyrimidin-2-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-2-yl, Tetrahydro-1,3-oxazin-4-yl, Tetrahydro-1,3-oxazin-5-yl, Tetrahydro-1,3-oxazin-6-yl, Tetrahydro-1,3-thiazin-2-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4- yl, Tetrahydro-1,2-oxazin-5-yl, Tetrahydro-1,2-oxazin-6-yl;
   C-gebundene, 6-gliedrige, partiell gesättigte Ringe, wie:
      2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-3,4-Dihydropyran-6-yl, 2H-3,4-Dihydrothiopyran-5-yl, 2H-3,4-Dihydrothiopyran-4-yl, 2H-3,4-Dihydropyran-3-yl, 2H-3,4-Dihydropyran-2-yl, 1,2,3,4-Tetrahydropyridin-6-yl, 1,2,3,4-Tetrahydropyridin-5-yl, 1,2,3,4-Tetrahydropyridin-4-yl, 1,2,3,4-Tetrahydropyridin-3-yl, 1,2,3,4-Tetrahydropyridin-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 2H-5,6-Dihydrothiopyran-2-yl, 2H-5,6-Dihydrothiopyran-3-yl, 2H-5,6-Dihydrothiopyran-4-yl, 2H-5,6-Dihydrothiopyran-5-yl, 2H-5,6-Dihydrothiopyran-6-yl, 1,2,5,6-Tetrahydropyridin-2-yl, 1,2,5,6-Tetrahydropyridin-3-yl, 1,2,5,6-Tetrahydropyridin-4-yl, 1,2,5,6-Tetrahydropyridin-5-yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl-, 4H-Pyran-4-yl, 4H-Thiopyran-2-yl, 4H-Thiopyran-3-yl, 4H-Thiopyran-4-yl, 1,4-Dihydropyridin-2-yl, 1,4-Dihydropyridin-3-yl, 1,4-Dihydropyridin-4-yl, 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 1,2-Dihydropyridin-2-yl, 1,2-Dihydropyridin-3-yl, 1,2-Dihydropyridin-4-yl, 1,2-Dihydropyridin-5-yl, 1,2-Dihydropyridin-6-yl, 3,4-Dihydropyridin-2-yl, 3,4-Dihydropyridin-3-yl, 3,4-Dihydropyridin-4-yl, 3,4-Dihydropyridin-5-yl, 3,4-Dihydropyridin-6-yl, 2,5-Dihydropyridin-2-yl, 2,5-Dihydropyridin-3-yl, 2,5-Dihydropyridin-4-yl, 2,5-Dihydropyridin-5-yl, 2,5-Dihydropyridin-6-yl, 2,3-Dihydropyridin-2-yl, 2,3-Dihydropyridin-3-yl, 2,3-Dihydropyridin-4-yl, 2,3-Dihydropyridin-5-yl, 2,3-Dihydropyridin-6-yl, 2H-5,6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5,6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1,2-oxazin-4-yl, 4H-5,6-Dihydro-1,2-oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3,6-Dihydro-1,2-oxazin-3-yl, 2H-3,6-Dihydro-1,2-oxazin-4-yl, 2H-3,6-Dihydro-1,2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Dihydro-1,2-thiazin-5-yl, 2H-3,6-Dihydro-1,2-thiazin-6-yl, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-1,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5-Tetrahydropyridazin-5-yl, 2,3,4,5-Tetrahydropyridazin-6-yl, 3,4,5,6-Tetrahydropyridazin-3-yl, 3,4,5,6-Tetrahydropyridazin-4-yl, 1,2,5,6-Tetrahydropyridazin-3-yl, 1,2,5,6-Tetrahydropyridazin-4-yl, 1,2,5,6-Tetrahydropyridazin-5-yl, 1,2,5,6-Tetrahydropyridazin-6-yl, 1,2,3,6-Tetrahydropyridazin-3-yl, 1,2,3,6-Tetrahydropyridazin-4-yl, 4H-5,6-Dihydro-1,3-oxazin-2-yl, 4H-5,6-Dihydro-1,3-oxazin-4-yl, 4H-5,6-Dihydro-1,3-oxazin-5-yl, 4H-5,6-Dihydro-1,3-oxazin-6-yl, 4H-5,6-Dihydro-1,3-thiazin-2-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5-6-Tetrahydropyrimidin-2-yl, 3,4,5,6-Tetrahydropyrimidin-4-yl, 3,4,5,6-Tetrahydropyrimidin-5-yl, 3,4,5,6-Tetrahydropyrimidin-6-yl, 1,2,3,4-Tetrahydropyrazin-2-yl, 1,2,3,4-Tetrahydropyrazin-5-yl, 1,2,3,4-Tetrahydropyrimidin-2-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl, 2H-1,2-Thiazin-6-yl, 4H-1,2-Oxazin-3-yl, 4H-1,2-Oxazin-4-yl, 4H-1,2-Oxazin-5-yl, 4H-2,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-Oxazin-3-yl, 6H-1,2-Oxazin-4-yl, 6H-1,2-Oxazin-5-yl, 6H-1,2-Oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-2-yl, 2H-1,3-Oxazin-4-yl, 2H-1,3-Oxazin-5-yl, 2H-1,3-Oxazin-6-yl, 2H-1,3-Thiazin-2-yl, 2H-1,3-Thiazin-4-yl, 2H-1,3-Thiazin-5-yl, 2H-1,3-Thiazin-6-yl, 4H-1,3-Oxazin-2-yl, 4H-1,3-Oxazin-4-yl, 4H-1,3-Oxazin-5-yl, 4H-1,3-Oxazin-6-yl, 4H-1,3-Thiazin-2-yl, 4H-1,3-Thiazin-4-yl, 4H-1,3-Thiazin-5-yl, 4H-1,3-Thiazin-6-yl, 6H-1,3-Oxazin-2-yl, 6H-1,3-Oxazin-4-yl, 6H-1,3-Oxazin-5-yl, 6H-1,3-Oxazin-6-yl, 6H-1,3-Thiazin-2-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl; 4H-1,4-Oxazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5-yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1,4-Dihydropyrimidin-2-yl, 1,4-Dihydropyrimidin-4-yl, 1,4-Dihydropyrimidin-5-yl, 1,4-Dihydropyrimidin-6-yl, 3,4-Dihydropyrimidin-2-yl, 3,4-Dihydropyrimidin-4-yl, 3,4-Dihydropyrimidin-5-yl oder 3,4-Dihydropyrimidin-6-yl;
   C-gebundene, 6-gliedrige, ungesättigte Ringe, wie:
      Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl, 1,2,4-Triazin-3-yl, 1,2,4-Triazin-5-yl, 1,2,4-Triazin-6-yl, 1,2,4,5-Tetrazin-3-yl;
   N-gebundene, 5-gliedrige, gesättigte Ringe, wie:
      Tetrahydropyrrol-1-yl, Tetrahydropyrazol-1-yl, Tetrahydroisoxazol-2-yl, Tetrahydroisothiazol-2-yl, Tetrahydroimidazol-1-yl, Tetrahydrooxazol-3-yl, Tetrahydrothiazol-3-yl;
   N-gebundene, 5-gliedrige, partiell gesättigte Ringe, wie:
      2,3-Dihydro-1H-pyrrol-1-yl, 2,5-Dihydro-1H-pyrrol-1-yl, 4,5-Dihydro-1H-pyrazol-1-yl, 2,5-Dihydro-1H-pyrazol-1-yl, 2,3-Dihydro-1H-pyrazol-1-yl, 2,5-Dihydroisoxazol-2-yl, 2,3-Dihydroisoxazol-2-yl, 2,5-Dihydroisothiazol-2-yl, 2,3-Dihydroisoxazol-2-yl, 4,5-Dihydro-1H-imidazol-1-yl, 2,5-Dihydro-1H-imidazol-1-yl, 2,3-Dihydro-1H-imidazol-1-yl, 2,3-Dihydrooxazol-3-yl, 2,3-Dihydrothiazol-3-yl, 1,2,4-Δ⁴-Oxadiazolin-2-yl, 1,2,4-Δ²-Oxadiazolin-4-yl, 1,2,4-Δ³-Oxadiazolin-2-yl, 1,3,4-Δ²-Oxadiazolin-4-yl, 1,2,4-Δ⁵-Thiadiazolin-2-yl, 1,2,4-Δ³-Thiadiazolin-2-yl, 1,2,4-Δ²-Thiadiazolin-4-yl, 1,3,4-Δ²-Thiadiazolin-4-yl, 1,2,3-Δ²-Triazolin-1-yl, 1,2,4-Δ²-Triazolin-1-yl, 1,2,4-Δ²-Triazolin-4-yl, 1,2,4-Δ³-Triazolin-1-yl, 1,2,4-Δ¹-Triazolin-4-yl;
   N-gebundene, 5-gliedrige, ungesättigte Ringe, wie:
      Pyrrol-1-yl, Pyrazol-1-yl, Imidazol-1-yl, 1,2,3-Triazol-1-yl, 1,2,4-Triazol-1-yl, Tetrazol-1-yl;
   N-gebundene, 6-gliedrige, gesättigte Ringe, wie:
      Piperidin-1-yl, Hexahydropyrimidin-1-yl, Hexahydropyrazin-1-yl, Hexahydropyridazin-1-yl, Tetrahydro-1,3-oxazin-3-yl, Tetrahydro-1,3-thiazin-3-yl, Tetrahydro-1,4-thiazin-4-yl, Tetrahydro-1,4-oxazin-4-yl, Tetrahydro-1,2-oxazin-2-yl;
sowie N-gebundene, 6-gliedrige, partiell gesättigte Ringe, wie: 1,2,3,4-Tetrahydropyridin-1-yl, 1,2,5,6-Tetrahydropyridin-1-yl, 1,4-Dihydropyridin-1-yl, 1,2-Dihydropyridin-1-yl, 2H-5,6-Dihydro-1,2-oxazin-2-yl, 2H-5,6-Dihydro-1,2-thiazin-2-yl, 2H-3,6-Dihydro-1,2-oxazin-2-yl, 2H-3,6-Dihydro-1,2-thiazin-2-yl, 2H-3,4-Dihydro-1,2-oxazin-2-yl, 2H-3,4-Dihydro-1,2-thiazin-2-yl, 2,3,4,5-Tetrahydropyridazin-2-yl, 1,2,5,6-Tetrahydropyridazin-1-yl, 1,2,5,6-Tetrahydropyridazin-2-yl, 1,2,3,6-Tetrahydropyridazin-1-yl, 3,4,5,6-Tetrahydropyrimidin-3-yl, 1,2,3,4-Tetrahydropyrazin-1-yl, 1,2,3,4-Tetrahydropyrimidin-1-yl, 1,2,3,4-Tetrahydropyrimidin-3-yl, 2,3-Dihydro-1,4-thiazin-4-yl, 2H-1,2-Oxazin-2-yl, 2H-1,2-Thiazin-2-yl, 4H-1,4-Oxazin-4-yl, 4H-1,4-Thiazin-4-yl, 1,4-Dihydropyridazin-1-yl, 1,4-Dihydropyrazin-1-yl, 1,2-Dihydropyrazin-1-yl, 1,4-Dihydropyrimidin-1-yl oder 3,4-Dihydropyrimidin-3-yl;
wobei gegebenenfalls der Schwefel der genannten Heterocyclen zu S=O oder S(=O)₂ oxidiert sein kann
und wobei mit einem ankondensierten Phenylring oder mit einem C₃-C₆-Carboxyclus oder mit einem weiteren 5- bis 6-gliedrigen Heterocyclus ein bicyclisches Ringsystem ausgebildet werden kann.

Die Phenylringe bzw. Heterocyclylreste sind vorzugsweise unsubstituiert oder tragen ein, zwei oder drei Halogenatome und/oder eine Nitrogruppe, eine Cyanogruppe, eine oder zwei Methyl-, Trifluormethyl-, Methoxy- oder Trifluormethoxygruppen.

In der Formel I steht (stehen) vorzugsweise
- R¹: für C₁-C₆-Alkyl, C₁-C₆-Halogenalkyl, C₁-C₆-Alkoxy; insbesondere C₁-C₆-Alkyl;
besonders bevorzugt C₁-C₄-Alkyl, wobei Methyl am stärksten bevorzugt ist;
- R²: für C₁-C₆-Halogenalkyl, C₁-C₆-Alkylsulfonyl, Halogen oder Nitro;
insbesondere C₁-C₆-Halogenalkyl, vorzugsweise Difluormethyl oder Trifluormethyl, C₁-C₆-Alkylsulfonyl oder Halogen, vorzugsweise Fluor oder Chlor;
besonders bevorzugt C₁-C₄-Alkylsulfonyl, wobei Methylsulfonyl und Ethylsulfonyl am stärksten bevorzugt sind;
- R³: für Wasserstoff, C₁-C₄-Alkyl oder Halogen;
insbesondere Wasserstoff, Methyl oder Chlor;
besonders bevorzugt Wasserstoff;
- R⁴: für C₁-C₂-Halogenalkyl;
insbesondere Fluormethyl, Chlormethyl, Brommethyl, Difluormethyl, Trifluormethyl, 1-Chlor-1-ethyl, 1-Fluor-1-ethyl oder Pentafluormethyl;
besonders bevorzugt Fluormethyl, Chlormethyl, Brommethyl, Difluormethyl und Trifluormethyl;
- R⁵: für Wasserstoff, C₁-C₂-Alkyl, C₁-C₂-Halogenalkyl;
insbesondere Wasserstoff, Methyl, Chlormethyl oder Trifluormethyl;
besonders bevorzugt Wasserstoff, Methyl oder Chlormethyl;
- R⁶: für Wasserstoff oder C₁-C₄-Alkyl;
insbesondere Wasserstoff oder Methyl;
- R⁷: für Wasserstoff oder C₁-C₄-Alkyl;
insbesondere Wasserstoff oder Methyl;
besonders bevorzugt Wasserstoff;
- R⁸: für Hydroxy, OR¹⁵, SR¹⁵, SOR¹⁶ oder SO₂R¹⁶;
insbesondere Hydroxy, OR¹⁵ oder SR¹⁵;
besonders bevorzugt Hydroxy;
- R⁹, R¹³: unabhängig voneinander für Wasserstoff, C₁-C₄-Alkyl oder C₁-C₄-Alkylthio;
insbesondere Wasserstoff, Methyl oder Methylthio;
besonders bevorzugt Wasserstoff;
- R¹⁰, R¹², R¹⁴: unabhängig voneinander für Wasserstoff oder Methyl;
- R¹¹: für Wasserstoff, Hydroxy, C₁-C₆-Alkyl oder Di(C₁-C₆-alkoxy)methyl;
insbesondere Wasserstoff oder C₁-C₄-Alkyl;
oder
- R⁹ und R¹⁰ oder R¹⁰ und R¹¹ oder R¹¹ und R¹⁴ oder R¹⁰ und R¹⁴ oder R¹³ und R¹⁴: gemeinsam für C₁-C₅-Alkandiyl, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
insbesondere R⁹ und R¹⁰ oder R¹⁰ und R¹⁴ gemeinsam für C₁-C₅-Alkandiyl;
oder
- R¹¹ und R¹²: gemeinsam für ein Sauerstoffatom;
- R¹⁵: für C₁-C₆-Alkyl, C₁-C₂₀-Alkylcarbonyl, vorzugsweise C₁-C₆-Alkylcarbonyl, C₁-C₆-Alkoxycarbonyl, C₁-C₆-Alkylthiocarbonyl, wobei die genannten Alkyl- und Alkoxyreste partiell oder vollständig halogeniert sein können und/oder einen, zwei oder drei unter Cyano, C₁-C₄-Alkaxy, C₁-C₄-Alkylthio, C₁-C₄-Alkylcarbonyl, C₁-C₄-Alkoxycarbonyl oder C₃-C₆-Cycloalkyl ausgewählte Substituenten tragen können;
Phenyl, Phenyl-C₁-C₄-alkyl, Phenylcarbonyl-C₁-C₄-alkyl, Phenylcarbonyl, Phenoxycarbonyl, Heterocyclyl; Heterocyclyl-C₁-C₄-alkyl, Heterocyclylcarbonyl-C₁-C₄-alkyl, Heterocyclyloxycarbonyl, wobei der Phenyl- oder Heterocyclylrest der genannten Reste partiell oder vollständig halogeniert sein kann und/oder einen, zwei oder drei unter Nitro, Cyano, C₁-C₄-Alkyl, C₁-C₄-Halogenalkyl, C₁-C₄-Alkoxy oder C₁-C₄-Halogenalkoxy ausgewählten Substituenten tragen kann.
- R¹⁶: C₁-C₆-Alkyl, das partiell oder vollständig halogeniert sein kann und/oder einen, zwei oder drei unter Cyano, C₁-C₄-Alkoxy, C₁-C₄-Alkylthio, C₁-C₄-Alkylcarbonyl, C₁-C₄-Alkoxycarbonyl oder C₃-C₆-Cycloalkyl ausgewählte Substituenten tragen kann; Phenyl, Phenyl-C₁-C₄-alkyl, Phenylcarbonyl-C₁-C₄-alkyl, Heterocyclyl, Heterocyclyl-C₁-C₄-alkyl oder Heterocyclylcarbonyl-C₁-C₄-alkyl, wobei der Phenyl- oder Heterocyclylrest der genannten Reste partiell oder vollständig halogeniert sein kann und/oder einen, zwei oder drei unter Nitro, Cyano, C₁-C₄-Alkyl, C₁-C₄-Halogenalkyl, C₁-C₄-Alkoxy oder C₁-C₄-Halogenalkoxy ausgewählten Substituenten tragen kann;

Insbesondere bevorzugt sind Halogenalkyl-substituierte 3-(4,5-Dihydroisoxazol-3-yl)-Benzoylcyclohexenone der Formel I, worin
- R⁴: für Fluormethyl, Chlormethyl, Brommethyl, Difluormethyl, Trifluormethyl, 1-Chlor-1-ethyl, 1-Fluor-1-ethyl oder Pentafluorethyl; besonders für Fluormethyl, Chlormethyl, Brommethyl, Difluormethyl oder Trifluormethyl steht.

Außerordentlich bevorzugt sind Halogenalkyl-substituierte 3-(4,5-Dihydroisoxazol-3-yl)-Benzoylcyclohexenone der Formel I, worin
- R¹: für C₁-C₄-Alkyl;
- R²: für C₁-C₆-Halogenalkyl, C₁-C₆-Alkylsulfonyl, Halogen oder Nitro; und
- R³: für Wasserstoff steht.

Besonders bevorzugt sind weiterhin Halogenalkyl-substituierte 3-(4,5-Dihydroisoxazol-3-yl)-Benzoylcyclohexenone der Formel I, worin
- R⁹ und R¹³: unabhängig voneinander für Wasserstoff, C₁-C₄-Alkyl oder C₁-C₄-Alkylthio;
- R¹⁰, R¹², R¹⁴: unabhängig voneinander für Wasserstoff oder Methyl stehen und
- R¹¹: für Wasserstoff, Hydroxy, C₁-C₆-Alkyl oder Di(C₁-C₆-alkoxy)methyl steht;
oder
- R⁹ und R¹⁰ oder R¹⁰ und R¹¹ oder R¹¹ und R¹⁴ oder R¹⁰ und R¹⁴ oder R¹³ und R¹⁴: gemeinsam für C₁-C₅-Alkandiyl stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
oder
- R¹¹ und R¹²: gemeinsam für ein Sauerstoffatom stehen.

Besonders bevorzugt sind weiterhin Halogenalkyl-substituierte 3-(4,5-Dihydroisoxazol-3-yl)-Benzoylcyclohexenone der Formel I, worin
- R⁸: für Hydroxy, OR¹⁵ oder SR¹⁵; und
- R¹⁵: für C₁-C₆-Alkyl, C₁-C₂₀-Alkylcarbonyl, C₁-C₆-Alkoxycarbonyl, C₁-C₆-Alkylthiocarbonyl, wobei die Alkyl- und Alkoxyreste partiell oder vollständig halogeniert sein können und/oder einen, zwei oder drei unter Cyano, C₁-C₄-Alkoxy, C₁-C₄-Alkylthio, C₁-C₄-Alkylcarbonyl, C₁-C₄-Alkoxycarbonyl oder C₃-C₆-Cycloalkyl ausgewählte Substituenten tragen können;
Phenyl, Phenyl-C₁-C₄-alkyl, Phenylcarbonyl-C₁-C₄-alkyl, Phenylcarbonyl, Phenoxycarbonyl, Heterocyclyl, Heterocyclyl-C₁-C₄-alkyl. Heterocyclylcarbonyl-C₁-C₄-alkyl, Heterocyclyloxycarbonyl steht, wobei der Phenyl- oder Heterocyclylrest der genannten Reste partiell oder vollständig halogeniert sein kann und/oder einen, zwei oder drei unter Nitro, Cyano, C₁-C₄-Alkyl, C₁-C₄-Halogenalkyl, C₁-C₄-Alkoxy oder C₁-C₄-Halogenalkoxy ausgewählten Substituenten tragen kann.
Heterocyclyl steht dabei vorzugsweise für einen C-gebundenen 5-gliedrigen ungesättigten Ring oder einen C-gebundenen 6-gliedrigen ungesättigten Ring, insbesondere Pyridin-2-yl oder Pyridin-3-yl.

Außerordentlich bevorzugt sind die Verbindungen der Formel Ia1 (≡I mit R³, R⁹ - R¹⁴ = H, R⁸ = OH), insbesondere die Verbindungen Ia1.1 bis Ia1.72 der Tabelle 1, wobei die Definitionen der Reste R¹ bis R¹⁴ nicht nur in Kombination miteinander, sondern auch jeweils für sich allein betrachtet für die erfindungsgemäßen Verbindungen eine besondere Bedeutung haben.

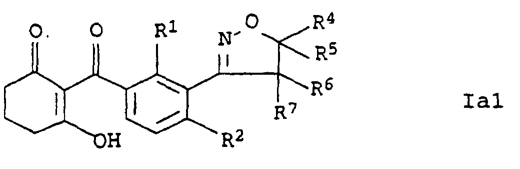

**Tabelle 1:**

| Nr. | R¹ | R² | R⁴ | R⁵ | R⁶ | R⁷ |
|---|---|---|---|---|---|---|
| Ia1.1 | CH₃ | SO₂CH₃ | CH₂F | H | H | H |
| Ia1.2 | CH₃ | SO₂CH₃ | CH₂F | CH₂F | H | H |
| Ia1.3 | CH₃ | SO₂CH₃ | CH₂F | CH₃ | H | H |
| Ia1.4 | CH₃ | SO₂CH₃ | CH₂Cl | H | H | H |
| Ia1.5 | CH₃ | SO₂CH₃ | CH₂Cl | CH₂Cl | H | H |
| Ia1.6 | CH₃ | SO₂CH₃ | CH₂Cl | CH₃ | H | H |
| Ia1.7 | CH₃ | SO₂CH₃ | CH₂Br | H | H | H |
| Ial.8 | CH₃ | SO₂CH₃ | CH₂Br | CH₃ | H | H |
| Ia1.9 | CH₃ | SO₂CH₃ | CHF₂ | H | H | H |
| Ia1.10 | CH₃ | SO₂CH₃ | CHF₂ | CH₃ | H | H |
| Ia1.11 | CH₃ | SO₂CH₃ | CHF₂ | CHF₂ | H | H |
| Ia1.12 | CH₃ | SO₂CH₃ | CF₃ | H | H | H |
| Ia1.13 | CH₃ | SO₂CH₃ | CF₃ | CF₃ | H | H |
| Ia1.14 | CH₃ | SO₂CH₃ | CF₃ | CH₃ | H | H |
| Ia1.15 5 | CH₃ | SO₂CH₃ | CHClCH₃ | H | H | H |
| Ia1.16 | CH₃ | SO₂CH₃ | CHBrCH₃ | H | H | H |
| Ia1.17 | CH₃ | SO₂CH₃ | CHFCH₃ | H | H | H |
| Ia1.18 | CH₃ | SO₂CH₃ | CF₂CF₃ | H | H | H |
| Ia1.19 | CH₃ | CF₃ | CH₂F | H | H | H |
| Ia1.20 | CH₃ | CF₃ | CH₂F | CH₂F | H | H |
| Ia1.21 | CH₃ | CF₃ | CH₂F | CH₃ | H | H |
| Ia1.22 2 | CH₃ | CF₃ | CH₂Cl | H | H | H |
| Ia1.23 | CH₃ | CF₃ | CH₂Cl | CH₂Cl | H | H |
| Ia1.24 | CH₃ | CF₃ | CH₂Cl | CH₃ | H | H |
| Ia1.25 | CH₃ | CF₃ | CH₂Br | H | H | H |
| Ia1.26 | CH₃ | CF₃ | CH₂Br | CH₃ | H | H |
| Ia1.27 | CH₃ | CF₃ | CHF₂ | H | H | H |
| Ia1.28 | CH₃ | CF₃ | CHF₂ | CH₃ | H | H |
| Ia1.29 | CH₃ | CF₃ | CHF₂ | CHF₂ | H | H |
| Ia1.30 | CH₃ | CF₃ | CF₃ | H | H | H |
| Ia1.31 | CH₃ | CF₃ | CF₃ | CF₃ | H | H |
| Ia1.32 | CH₃ | CF₃ | CF₃ | CH₃ | H | H |
| Ia1.33 | CH₃ | CF₃ | CHCICH₃ | H | H | H |
| Ia1.34 | CH₃ | CF₃ | CHBrCH₃ | H | H | H |
| Ia1.35 | CH₃ | CF₃ | CHFCH₃ | H | H | H |
| Ia1.36 | CH₃ | CF₃ | CF₂CF₃ | H | H | H |
| Ia1.37 | CH₂CH₃ | SO₂CH₃ | CH₂F | H | H | H |
| Ia1.38 | CH₂CH₃ | SO₂CH₃ | CH₂F | CH₂F | H | H |
| Ia1.39 | CH₂CH₃ | SO₂CH₃ | CH₂F | CH₃ | H | H |
| Ia1.40 | CH₂CH₃ | SO₂CH₃ | CH₂Cl | H | H | H |
| Ia1.41 | CH₂CH₃ | SO₂CH₃ | CH₂Cl | CH₂Cl | H | H |
| Ia1.42 | CH₂CH₃ | SO₂CH₃ | CH₂Cl | CH₃ | H | H |
| Ia1.43 | CH₂CH₃ | SO₂CH₃ | CH₂Br | H | H | H |
| Ia1.44 | CH₂CH₃ | SO₂CH₃ | CH₂Br | CH₃ | H | H |
| Ia1.45 | CH₂CH₃ | SO₂CH₃ | CHF₂ | H | H | H |
| Ia1.46 | CH₂CH₃ | SO₂CH₃ | CHF₂ | CH₃ | H | H |
| Ia1.47 | CH₂CH₃ | SO₂CH₃ | CHF₂ | CHF₂ | H | H |
| Ia1.48 | CH₂CH₃ | SO₂CH₃ | CF₃ | H | H | H |
| Ia1.49 | CH₂CH₃ | SO₂CH₃ | CF₃ | CF₃ | H | H |
| Ia1.50 | CH₂CH₃ | SO₂CH₃ | CF₃ | CH₃ | H | H |
| Ia1.51 | CH₂CH₃ | SO₂CH₃ | CHClCH₃ | H | H | H |
| Ia1.52 | CH₂CH₃ | SO₂CH₃ | CHBrCH₃ | H | H | H |
| Ia1.53 | CH₂CH₃ | SO₂CH₃ | CHFCH₃ | H | H | H |
| Ia1.54 | CH₂CH₃ | SO₂CH₃ | CF₂CF₃ | H | H | H |
| Ia1.55 | CH₃ | SO₂CH₂CH₃ | CH₂F | H | H | H |
| Ia1.56 | CH₃ | SO₂CH₂CH₃ | CH₂F | CH₂F | H | H |
| Ia1.57 | CH₃ | SO₂CH₂CH₃ | CH₂F | CH₃ | H | H |
| Ia1.58 | CH₃ | SO₂CH₂CH₃ | CH₂Cl | H | H | H |
| Ia1.59 | CH₃ | SO₂CH₂CH₃ | CH₂Cl | CH₂Cl | H | H |
| Ia1.60 | CH₃ | SO₂CH₂CH₃ | CH₂Cl | CH₃ | H | H |
| Ia1.61 | CH₃ | SO₂CH₂CH₃ | CH₂Br | H | H | H |
| Ia1.62 | CH₃ | SO₂CH₂CH₃ | CH₂Br | CH₃ | H | H |
| Ia1.63 | CH₃ | SO₂CH₂CH₃ | CHF₂ | H | H | H |
| Ia1.64 | CH₃ | SO₂CH₂CH₃ | CHF₂ | CH₃ | H | H |
| Ia1.65 | CH₃ | SO₂CH₂CH₃ | CHF₂ | CHF₂ | H | H |
| Ia1.66 | CH₃ | SO₂CH₂CH₃ | CF₃ | H | H | H |
| Ia1.67 | CH₃ | SO₂CH₂CH₃ | CF₃ | CF₃ | H | H |
| Ia1.68 | CH₃ | SO₂CH₂CH₃ | CF₃ | CH₃ | H | H |
| Ia1.69 | CH₃ | SO₂CH₂CH₃ | CHClCH₃ | H | H | H |
| Ia1.70 | CH₃ | SO₂CH₂CH₃ | CHBrCH₃ | H | H | H |
| Ia1.71 | CH₃ | SO₂CH₂CH₃ | CHFCH₃ | H | H | H |
| Ia1.72 | CH₃ | SO₂CH₂CH₃ | CF₂CF₃ | H | H | H |

Ebenso außerordentlich bevorzugt sind die Verbindungen der Formel Ia2, insbesondere die Verbindungen Ia2.1 bis Ia2.72, die sich von den entsprechenden Verbindungen Ia1.1 bis Ia1.72 dadurch unterscheiden, dass R¹¹ für Methyl steht.

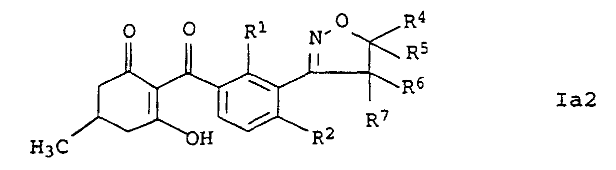

Ebenso außerordentlich bevorzugt sind die Verbindungen der Formel Ia3, insbesondere die Verbindungen Ia3.1 bis Ia3.72, die sich von den Verbindungen Ia1.1 bis Ia1.72 dadurch unterscheiden, dass R¹¹ und R¹² für Methyl stehen.

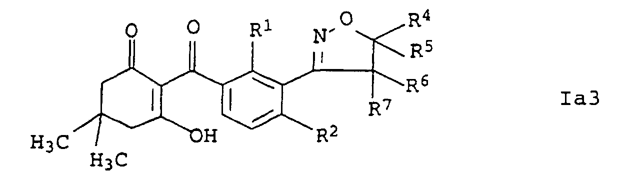

Ebenso außerordentlich bevorzugt sind die Verbindungen der Formel Ia4, insbesondere die Verbindungen Ia4.1 bis Ia4.72, die sich von den Verbindungen Ia1.1 bis Ia1.72 dadurch unterscheiden, dass R⁹ und R¹³ für Methyl stehen.

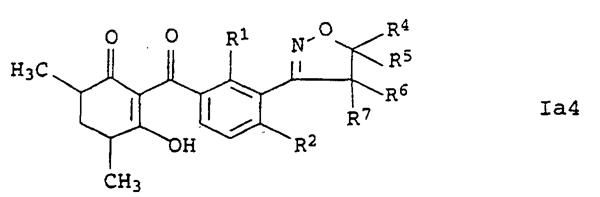

Ebenso außerordentlich bevorzugt sind die Verbindungen der Formel Ia5, insbesondere die Verbindungen Ia5.1 bis Ia5.72, die sich von den Verbindungen Ia1.1 bis Ia1.72 dadurch unterscheiden, dass R⁹ für Methylthio und R¹⁰ für Methyl stehen.

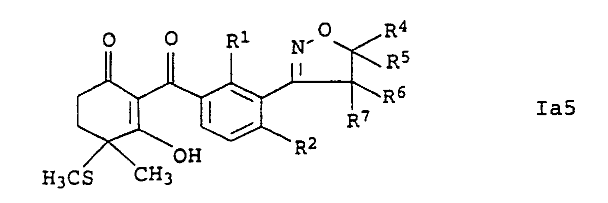

Ebenso außerordentlich bevorzugt sind die Verbindungen der Formel Ia6, insbesondere die Verbindungen Ia6.1 bis Ia6.72, die sich von den Verbindungen Ia1.1 bis Ia1.72 dadurch unterscheiden, dass R⁹ und R¹⁰ für gemeinsam für Pentan-1,5-diyl stehen.

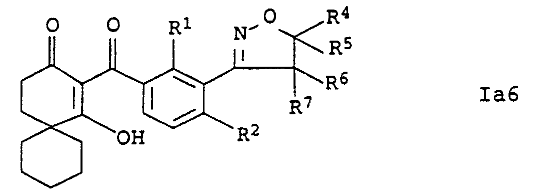

Ebenso außerordentlich bevorzugt sind die Verbindungen der Formel Ia7, insbesondere die Verbindungen Ia7.1 bis Ia7.72, die sich von den Verbindungen Ia1.1 bis Ia1.72 dadurch unterscheiden, dass R¹⁰ und R¹⁴ gemeinsam für Ethan-1,2-diyl stehen.

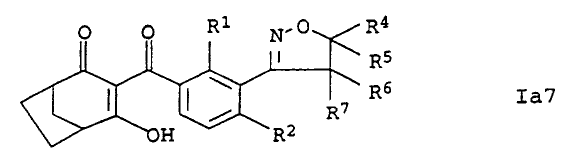

Ebenso außerordentlich bevorzugt sind die Verbindungen der Formel Ia8, insbesondere die Verbindungen Ia8.1 bis Ia8.72, die sich von den Verbindungen Ia1.1 bis Ia1.72 dadurch unterscheiden, dass R⁹, R¹⁰, R¹³ und R¹⁴ für Methyl und R¹¹ und R¹² gemeinsam für ein Sauerstoffatom stehen.

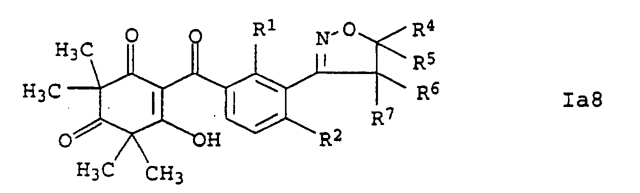

Ebenso außerordentlich bevorzugt sind die Verbindungen der Formel Ia9, insbesondere die Verbindungen Ia9.1 bis Ia9.72, die sich von den Verbindungen Ia1.1 bis Ia1.72 dadurch unterscheiden, dass R¹¹ für Hydroxy steht.

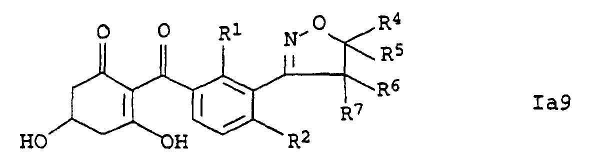

Die Halogenalkyl-substituierten 3-(4,5-Dihydroisoxazol-3-yl)-Benzoylcyclohexenone der Formel I, sind auf verschiedene Art und weise erhältlich, beispielsweise nach folgenden Verfahren.

### Verfahren A:

Durch Umsetzung von Verbindungen der Formel II mit einem aktivierten Benzoesäure-Derivat IIIα oder einer Benzoesäure IIIβ, die vorzugsweise in situ aktiviert wird, zu dem entsprechenden Acylierungsprodukt I' und anschließende Umlagerung erhält man Verbindungen der Formel I mit R⁸ = OH.

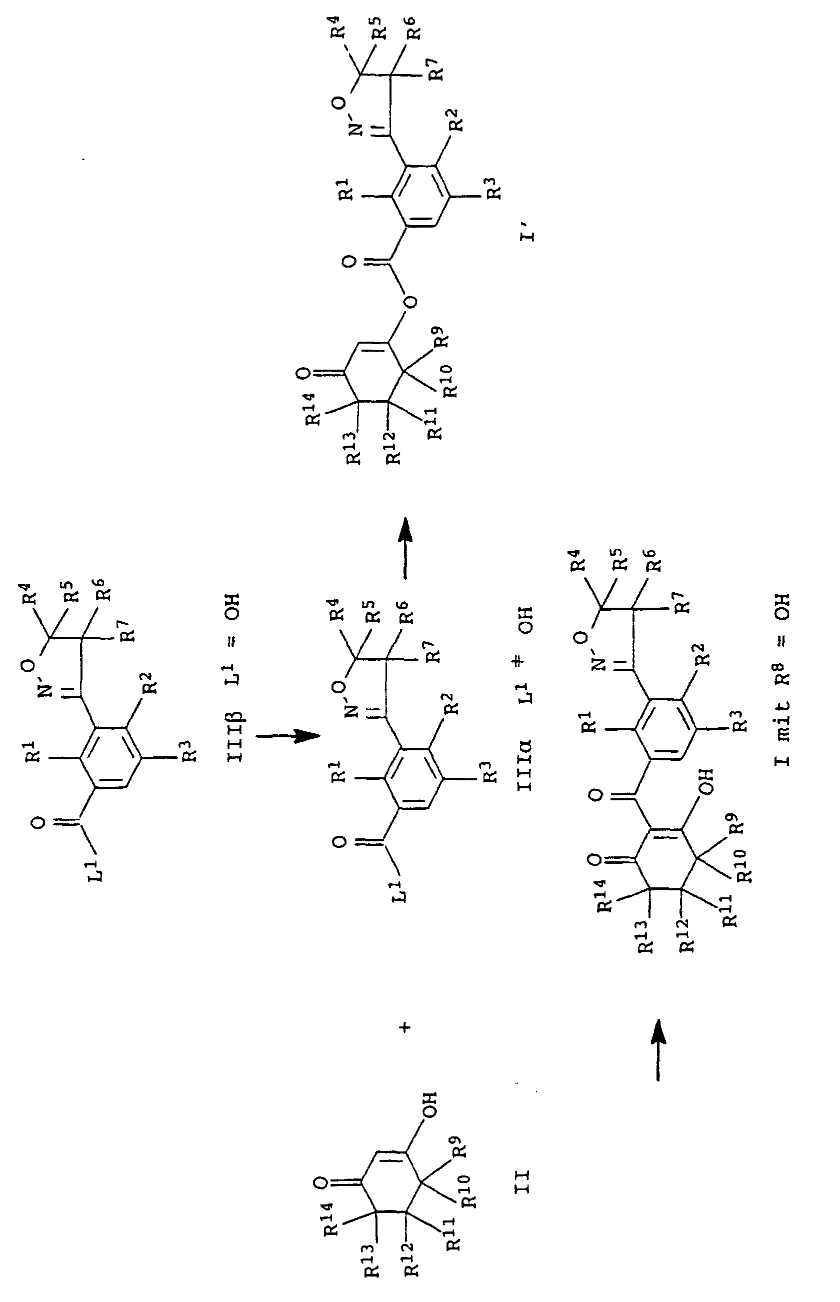

L¹ steht für Hydroxy oder eine nucleophil verdrängbare Abgangsgruppe, wie Halogen z. B. Brom, Chlor, Hetaryl, z. B. Imidazolyl, Pyridyl, Carboxylat, z. B. Acetat, Trifluoracetat, etc.

Die aktivierte Benzoesäure IIIa kann direkt eingesetzt werden, wie im Fall der Benzoylhalogenide oder in situ erzeugt werden, z. B. mit Dicyclohexylcarbodiimid, Triphenylphosphan/Azodicarbonsäureester, 2-Pyridindisulfid/Triphenylphosphan, Carbonyldiimidazol, etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z. B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z. B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Dimethoxyethan, Tetrahydrofuran, Dioxan, polare aprotische Lösungsmittel, wie Acetonitril, Dimethylformamid, Dimethylsulfoxid oder Ester, wie Essigsäureethylester oder Gemische hiervon verwendet werden.

Werden Benzoylhalogenide als aktivierte Carbonsäurekomponente eingesetzt, so kann es zweckmäßig sein, bei Zugabe dieses Reaktionspartners die Reaktionsmischung auf 0 - 10 °C abzukühlen. Anschließend rührt man bei 20 - 100 °C, vorzugsweise bei 25 - 50 °C, bis die Umsetzung vollständig ist. Die Aufarbeitung erfolgt in üblicher Weise, z. B. wird das Reaktionsgemisch auf Wasser gegossen, das Wertprodukt extrahiert. Als Lösungsmittel eignen sich hierfür besonders Methylenchlorid, Diethylether, Dimethoxyethan und Essigsäureethylester. Nach Trocknen der organischen Phase und Entfernen des Lösungsmittels kann der rohe Ester I' ohne weitere Reinigung zur Umlagerung eingesetzt werden.

Die Umlagerung der Ester I' zu den Verbindungen der Formel I erfolgt zweckmäßigerweise bei Temperaturen von 20 bis 40 °C in einem Lösungsmittel und in Gegenwart einer Base sowie gegebenenfalls mit Hilfe einer Cyanoverbindung als Katalysator.

Als Lösungsmittel können z. B. Acetonitril, Methylenchlorid, 1,2-Dichlorethan, Dioxan, Essigsäureethylester, Dimethoxyethan, Tetrahydrofuran, Toluol oder Gemische hiervon verwendet werden. Bevorzugte Lösungsmittel sind Acetonitril und Dioxan.

Geeignete Basen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuss, bezogen auf den Ester, eingesetzt werden. Bevorzugt werden Triethylamin oder Alkalicarbonate verwendet, vorzugsweise in doppelt äquimolaren Verhältnis in Bezug auf den Ester.

Als Cyanoverbindungen kommen anorganische Cyanide, wie Natriumcyanid, Kaliumcyanid und organische Cyanoverbindungen, wie Acetoncyanhydrin, Trimethylsilycyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z. B. in einer Menge von 5 bis 15, vorzugsweise 10 Molprozent, bezogen auf den Ester, eingesetzt. (Beispiele für die cyanidkatalysierte Umlagerung von Enolestern der Cyclohexan-1,3-dione sind z. B. in der EP-A 186 118 und der US 4,780,127 genannt).

Die Aufarbeitung kann in an sich bekannter Weise erfolgen. Das Reaktionsgemisch wird z. B. mit verdünnter Mineralsäure, wie 5 %ige Salzsäure oder Schwefelsäure, angesäuert, mit einem organischen Lösungsmittel, z. B. Methylenchlorid, Essigsäureethylester extrahiert. Der organische Extrakt kann mit 5-10 %iger Alkalicarbonatlösung, z. B. Natriumcarbonat-, Kaliumcarbonatlösung extrahiert werden. Die wässrige Phase wird angesäuert und der sich bildende Niederschlag abgesaugt und/oder mit Methylenchlorid oder Essigsäureethylester extrahiert, getrocknet und eingeengt.

Die Cyclohexenone der Formel II sind bekannt oder können nach an sich bekannten Verfahren hergestellt werden (z. B. EP-A 71 707, EP-A 142 741, EP-A 243 313, US 4,249,937, WO 92/13821).

Die Verbindungen der Formel IIIβ sind z. B. wie folgt erhältlich:

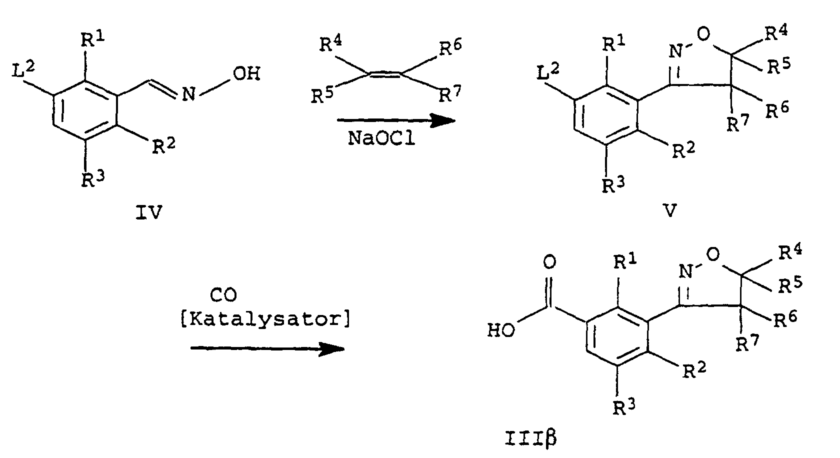

L² steht für eine Abgangsgruppe wie Halogen, z. B. Chlor, Brom oder Iod, oder Sulfonat wie Mesylat oder Triflat; bevorzugt sind Brom oder Triflat.

Die 4,5-Dihydroisoxazol-3-yl-benzolderivate V sind z. B. durch Umwandlung von Oximen der Formel IV in an sich bekannter Weise über die Zwischenstufe der Hydroxamsäurechloride erhältlich. Aus letzteren werden in situ Nitriloxide erzeugt, die mit Alkenen zu den gewünschten Produkten abreagieren (vgl. z. B. Chem. Ber. 106, 3258-3274 (1972)).

Das 4,5-Dihydroisoxazol-3-yl-benzolderivat V wird dann in Gegenwart eines Katalysators sowie einer Base mit Kohlenmonoxid und Wasser zu IIIβ umgesetzt.

Als Katalysatoren eignen sich Palladiumligandkomplexe, in denen das Palladium in der Oxidationsstufe 0 vorliegt, metallisches Palladium, das gegebenenfalls auf einen Träger aufgezogen wurde, und vorzugsweise Palladium(II)salze. Die Umsetzung mit Palladium(II)salzen und metallischem Palladium-wird vorzugsweise in Gegenwart von Komplexliganden durchgeführt.

Als Palladium(0)ligandkomplex kommt beispielsweise Tetrakis(triphenylphosphan)palladium in Frage.

Metallisches Palladium ist vorzugsweise auf einen inerten Träger wie beispielsweise Aktivkohle, Siliciumdioxid, Aluminiumoxid, Bariumsulfat oder Calciumcarbonat aufgezogen. Die Reaktion wird vorzugsweise in Gegenwart von Komplexliganden wie beispielsweise Triphenylphosphan durchgeführt.

Als Palladium (II) salze eigenen sich beispielsweise Palladiumacetat und Palladiumchlorid. Bevorzugt wird in Gegenwart von Komplexliganden wie beispielsweise Triphenylphosphan gearbeitet.

Geeignete Komplexliganden für die Palladiumligandkomplexe, bzw. in deren Gegenwart die Umsetzung mit metallischem Palladium oder Palladium(II)salzen vorzugsweise ausgeführt wird, sind tertiäre Phosphane, deren Struktur durch folgende Formeln wiedergegeben wird: 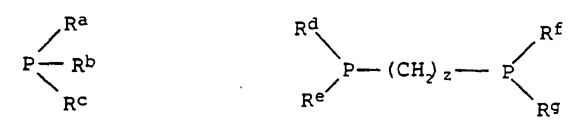 wobei z die Zahlen 1 bis 4 bedeutet und die Reste R^{a} bis R^{g} für C₁-C₆-Alkyl, C₃-C₆-Cycloalkyl, Aryl-C₁-C₂-alkyl oder vorzugsweise Aryl stehen. Aryl steht beispielsweise für Naphthyl und gegebenenfalls substituiertes Phenyl wie beispielsweise 2-Tolyl und insbesondere für unsubstituiertes Phenyl.

Die Herstellung der komplexen Palladiumsalze kann in an sich bekannter Weise ausgehend von kommerziell erhältlichen Palladiumsalzen wie Palladiumchlorid oder Palladiumacetat und den entsprechenden Phosphanen wie z. B. Triphenylphosphan oder 1,2-Bis(diphenylphosphano)ethan erfolgen. Ein Großteil der komplexierten Palladiumsalze ist auch kommerziell erhältlich. Bevorzugte Palladiumsalze sind [(R)(+)2,2'-Bis(diphenylphosphano)-1,1'-binaphthyl]palladium(II)chlorid, Bis(triphenylphosphan)palladium(II)acetat und insbesondere Bis(triphenylphosphan)palladium(II)chlorid.

Der Palladiumkatalysator wird in der Regel in einer Konzentration von 0,05 bis 5 Mol-%, bevorzugt 1 bis 3 Mol-%, eingesetzt.

Als Basen kommen tertiäre Amine, wie beispielsweise N-Methylpiperidin, Ethyldiisopropylamin, 1,8-Bisdimethylaminonaphthalin oder insbesondere Triethylamin in Betracht. Ebenso eigenen sich Alkalicarbonat, wie Natriumcarbonat oder Kaliumcarbonat. Aber auch Gemische von Kaliumcarbonat und Triethylamin sind geeignet.

In der Regel werden 2 bis 4 Moläquivalente, insbesondere 2 Moläquivalente, des Alkalicarbonats, sowie 1 bis 4 Moläquivalente, insbesondere 2 Moläquivalente des tertiären Amins bezogen auf das 4,5-Dihydroisoxazol-3-yl-benzolderivat der Formel V eingesetzt.

Als Lösungsmittel können Nitrile, wie Benzonitril und Acetonitril, aromatische Kohlenwasserstoff, wie Toluol, Amide, wie Dimethylformamid, Dimethylacetamid, Tetra-C₁-C₄-alkylharnstoffe oder N-Methylpyrrolidon und vorzugsweise Ether, wie Tetrahydrofuran, Methyl-tert.-butylether, dienen. Insbesondere werden Ether, wie 1,4-Dioxan und Dimethoxyethan, als Lösungsmittel bevorzugt.

Ebenso ist es möglich, die Verbindungen der Formel IIIα (L¹ = OH) zu erhalten, indem man ein Oxim der Formel VI in das entsprechende Hydroxamsäurehalogenid, insbesondere Hydroxamsäurechlorid, überführt, in situ ein Nitriloxid erzeugt und dieses mit einem Alken umsetzt (vgl. z. B. Chem. Ber. 106, 3258-3274 (1973)). Anschließend wird der Ester VII unter an sich bekannten Bedingungen verseift zu den Verbindungen der Formel III mit L¹ = Hydroxy. 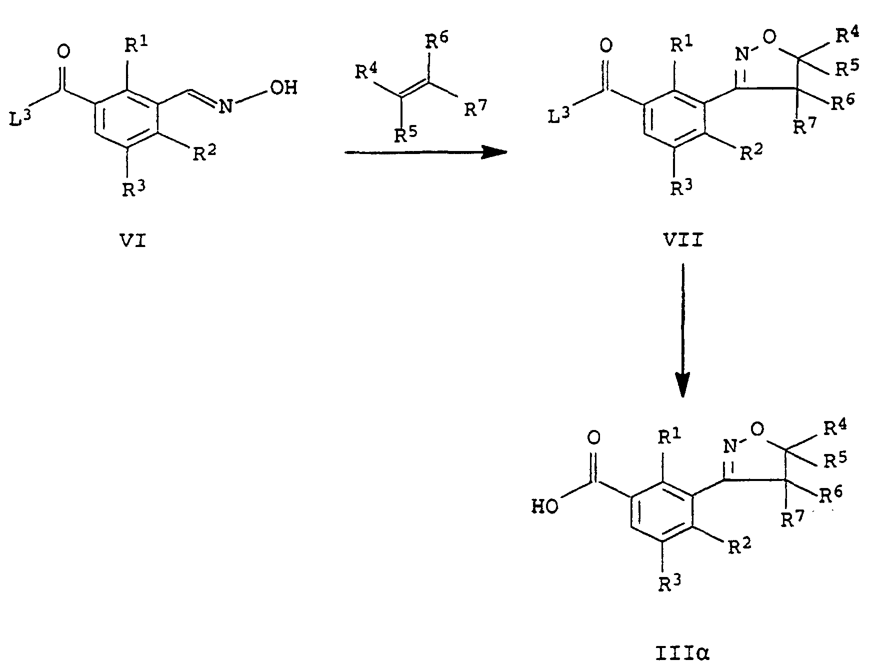 L³ steht für einen C₁-C₆-Alkoxy-Rest.

Verbindungen der Formel I mit R⁸ = OR¹⁵ bzw. SR¹⁵ werden durch Umsetzung von Verbindungen der Formel I mit R⁸ = Hydroxy bzw. Mercapto mit Alkylierungsmitteln, Carbamoylierungsmittel bzw. Acylierungsmitteln L⁴-R¹⁵ (IV) erhalten.

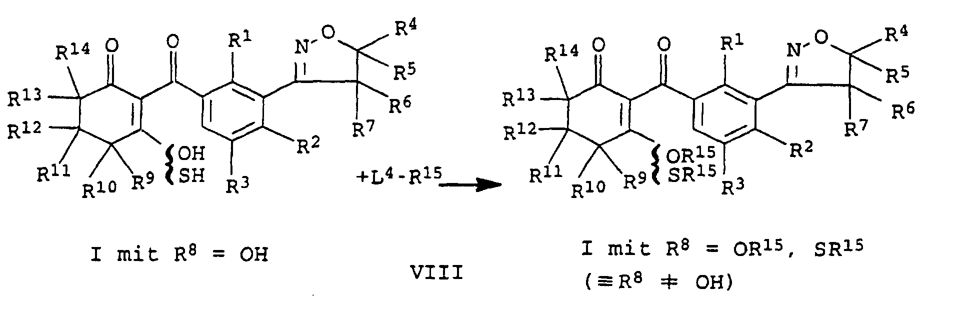

L⁴ steht für eine nucleophil verdrängbare Abgangsgruppe, wie Halogen, z. B. Brom oder Chlor, Acyloxy, z. B. Acetyloxy, Ethylcarbonyloxy, oder Alkylsulfonyloxy, z. B. Methylsulfonyloxy oder Trifluormethylsulfonyloxy;

Die Verbindungen der Formel VIII können direkt eingesetzt werden, wie z. B. im Fall der Carbonsäurehalogenide, Carbonsäureanhydride oder in situ erzeugt werden (z. B. mittels Dicyclohexylcarbonyldiimid, Carbonyldiimidazol etc.).

Die Ausgangsverbindungen werden in der Regel im äquimolaren Verhältnis eingesetzt. Es kann aber auch von Vorteil sein, die eine oder andere Komponente im Überschuss einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuss der Hilfsbase z. B. 1,5 bis 3 Moläquivalente, bezogen auf I, kann unter Umständen vorteilhaft sein.

Als Hilfsbasen eignen sich tertiäre Alkylamine, wie Triethylamin, aromatische Amine, wie Pyridin, Alkalimetallcarbonate, z. B. Natriumcarbonat oder Kaliumcarbonat, Alkalimetallhydrogencarbonate, wie Natriumhydrogencarbonat oder Kaliumhydrogencarbonat, Alkalimetallalkoholate, wie Natriummethanolat, Natriumethanolat, Kalium-tert-butylat oder Alkalimetallhydride, z. B. Natriumhydrid. Bevorzugt verwendet werden Triethylamin und Pyridin.

Als Lösungsmittel kommen z. B. chlorierte Kohlenwasserstoffe, wie Methylenchlorid, 1,2-Dichlorethan, aromatische Kohlenwasserstoffe, z. B. Toluol, Xylol, Chlorbenzol, Ether, wie Diethylether, Methyl-tert-butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0 °C bis zur Höhe des Siedepunktes des Reaktionsgemisches.

Die Aufarbeitung kann in an sich bekannter Weise zum Produkt hin erfolgen.

Verbindungen der Formel I mit R⁸ = Halogen werden durch Umsetzung von Verbindungen der Formel I mit R⁸ = Hydroxy mit einem Halogenierungsmittel erhalten (Hal steht für Halogen).

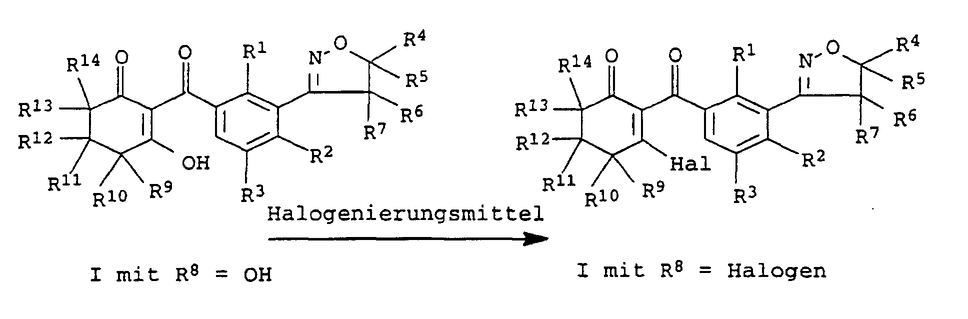

Als Halogenierungsmittel eignen sich beispielsweise Phosgen, Diphosgen, Triphosgen, Thionylchlorid, Oxalylchlorid, Phosphoroxychlorid, Phosphorpentachlorid, Mesylchlorid, Chlormethylen-N,N-dimethylammoniumchlorid, Oxalylbromid, Phosphoroxybromid etc.

Die Ausgangsverbindungen werden in der Regel im äquimolaren Verhältnis eingesetzt. Es kann auch von Vorteil sein, die eine oder andere Komponente im Überschuss einzusetzen.

Als Lösungsmittel kommen z. B. chlorierte Kohlenwasserstoffe, wie Methylenchlorid oder 1,2-Dichlorethan, aromatische Kohlenwasserstoffe, z. B. Toluol, Xylol oder Chlorbenzol, polare aprotische Lösungsmittel, wie Acetonitril, Dimethylformamid oder Dimethylsulfoxid oder Gemische hiervon in Betracht. Es ist auch möglich, die Reaktion in Substanz durchzuführen.

In der Regel liegt die Reaktionstemperatur im Bereich von 0 °C bis zur Höhe des Siedepunktes des Reaktionsgemisches.

Die Aufarbeitung kann in an sich bekannter Weise zum Produkt hin erfolgen.

Verbindungen der Formel I mit R⁸ = Mercapto, OR¹⁵ oder SR¹⁵ können durch Umsetzung von Verbindungen der Formel I mit R⁸ = Halogen mit Verbindungen IX, gegebenenfalls in Gegenwart einer Base oder unter vorangegangener Salzbildung erhalten werden.

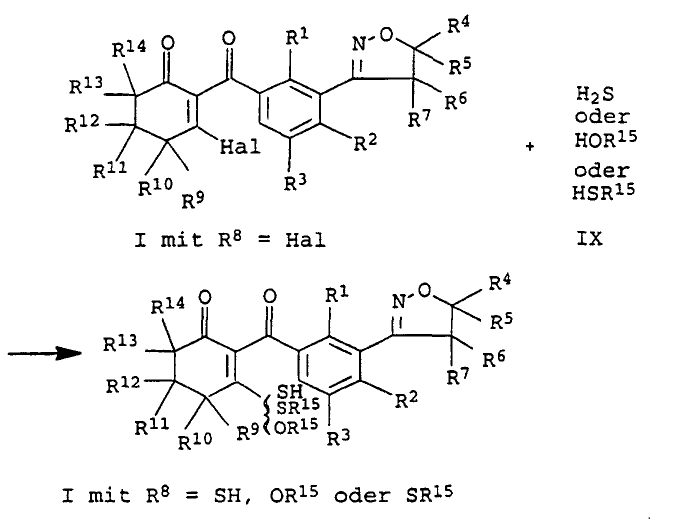

Die Ausgangsverbindungen werden in der Regel im äquimolaren Verhältnis eingesetzt. Es kann aber auch von Vorteil sein, die eine oder andere Komponente im Überschuss einzusetzen.

Gegebenenfalls kann es auch von Vorteil sein, die Umsetzung in Gegenwart einer Base durchzuführen. Die Reaktanden und die Base werden dabei zweckmäßigerweise in äquimolaren Mengen eingesetzt. Ein Überschuss an Base, von z. B. 1,5 bis 3 Moläquivalente, bezogen auf I mit R⁸ = Hal, kann unter Umständen von Vorteil sein.

Als Basen eignen sich tertiäre Alkylamine, wie Triethylamin, aromatische Amine, wie Pyridin, Alkalimetallcarbonate, z. B. Natriumcarbonat oder Kaliumcarbonat, Alkalimetallhydrogencarbonate, wie Natriumhydrogencarbonat oder Kaliumhydrogencarbonat, Alkalimetallalkoholate, wie Natriummethanolat, Natriumethanolat, Kalium-tert-butanolat oder Alkalimetallhydride, wie z. B. Natriumhydrid. Bevorzugt verwendet man Natriumhydrid oder Kalium-tert-butanolat.

Als Lösungsmittel kommen z. B. chlorierte Kohlenwasserstoffe, wie Methylenchlorid oder 1,2-Dichlorethan, aromatische Kohlenwasserstoffe, z. B. Toluol, Xylol oder Chlorbenzol, Ether, wie Diethylether, Methyl-tert-butylether, Tetrahydrofuran oder Dioxan, polare aprotische Lösungsmittel, wie Acetonitril, Dimethylformamid oder Dimethylsulfoxid oder Gemische hiervon in Betracht.

In der Regel liegt die Reaktionstemperatur im Bereich von 0 °C bis zur Höhe des Siedepunktes des Reaktionsgemisches.

Die Aufarbeitung kann in an sich bekannter Weise zum Produkt hin erfolgen.

Weiterhin können Verbindungen der Formel I mit R⁸ = SOR¹⁶ oder SO₂R¹⁶ mit einem Oxidationsmittel erhalten werden.

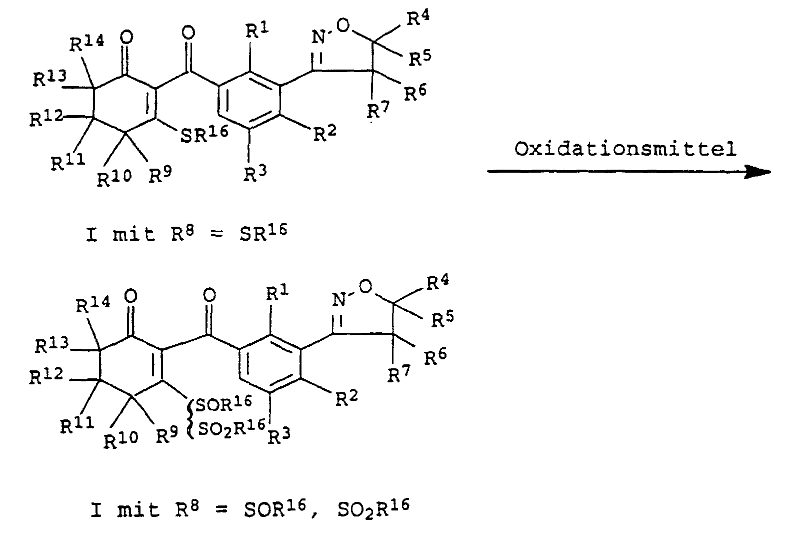

Als Oxidationsmittel kommen beispielsweise m-Chlorperbenzoesäure, Peroxyessigsäure, Trifluorperoxyessigsäure, Wasserstoffperoxid, gegebenenfalls in Gegenwart eines Katalysators, wie Wolframat, in Betracht.

Die Ausgangsverbindungen werden in der Regel in äquimolarem Verhältnis eingesetzt. Es kann von Vorteil sein, die eine oder andere Komponente im Überschuss einzusetzen.

Als Lösungsmittel kommen z. B. chlorierte Kohlenwasserstoffe, wie Methylenchlorid oder 1,2-Dichlorethan, aromatische Kohlenwasserstoffe, z. B. Toluol, Xylol oder Chlorbenzol, Ether, wie Diethylether, Methyl-tert-butylether, Tetrahydrofuran oder Dioxan, polare aprotische Lösungsmittel, wie Acetonitril oder Dimethylformamid oder Ester, wie Essigsäureethylester, oder Gemische hiervon in Betracht.

In der Regel liegt die Reaktionstemperatur im Bereich von 0 °C bis zur Höhe des Siedepunktes des Reaktionsgemisches.

Die Aufarbeitung kann in an sich bekannter Weise zum Produkt hin erfolgen.

### Herstellungsbeispiele

### 2-Methyl-3-(5-chlormethyl-4,5-dihydroisoxazol-3-yl)-4-methylsulfonylbenzoesäurechlorid

Zu einem Gemisch von 4,5 g (13,6 mmol) 2-Methyl-3-(5-chlormethyl-4,5-dihydroisoxazol-3-yl)-4-methylsulfonylbenzoesäure, 100 ml Toluol und drei Tropfen N,N-Dimethylformamid wurden 3,23 g (27,2 mmol) Thionylchlorid zugetropft. Nach 4 Stunden Erhitzen unter Rückfluss wurde abgekühlt und das Lösungsmittel entfernt. Anschließend wurde der Rückstand im Methylenchlorid aufgenommen und nochmals bis zur Trockne eingeengt. Man erhielt 4,74 g (100 % d. Th.) der Titelverbindung in Form eines hellen Pulvers.

### 3-[2-Methyl-3-(5-chlormethyl-4,5-dihydroisoxazol-3-yl)-4-methylsulfonyl-benzoyl]-bicyclo[3.2.1]octan-2,4-dion (Verbindung 2.2)

Zu 0,28 g (2 mmol) Bicyclo[3.2.1]octan-2,4-dion und 0,4 g (4 mmol) Triethylamin in 40 ml Acetonitril wurde unter Stickstoff bei 0 bis 5 °C 0,7 g (2 mmol) 2-Methyl-3-(5-chlormethyl-4,5-dihydroisoxazol-3-yl)-4-methylsulfonylbenzoylchlorid in 40 ml Acetonitril zugetropft und 4 Stunden bei Raumtemperatur gerührt. Anschließend wurden 1 Tropfen Trimethylsilylnitril und 0,05 g Kaliumcarbonat zugegeben und 2 Stunden bei 40 °C gerührt. Nach 12 Stunden Rühren bei Raumtemperatur wurde das Lösungsmittel entfernt, der Rückstand im Essigsäureethylester aufgenommen und mehrmals mit 5 %iger Kaliumcarbonat-Lösung extrahiert. Die vereinigten Extrakte wurden dann mit 10 %iger Salzsäure auf einen pH-Wert von 2 bis 3 eingestellt und mehrmals mit Methylenchlorid extrahiert. Diese vereinigten organischen Extrakte wurden nunmehr mit Wasser gewaschen, getrocknet und das Lösungsmittel entfernt. Der ölige, gelbe Rückstand wurde an Kieselgel (Eluent: Methylenchlorid/Methanol = 99/1 bis 97/3) chromatographiert. Man erhielt 0,4 g (44 % d. Th.) der Titelverbindung mit einem Schmelzpunkt von 98 - 103°C.

In Tabelle 2 sind Verbindungen der Formel I aufgeführt, die in analoger Weise hergestellt wurden oder herstellbar sind.

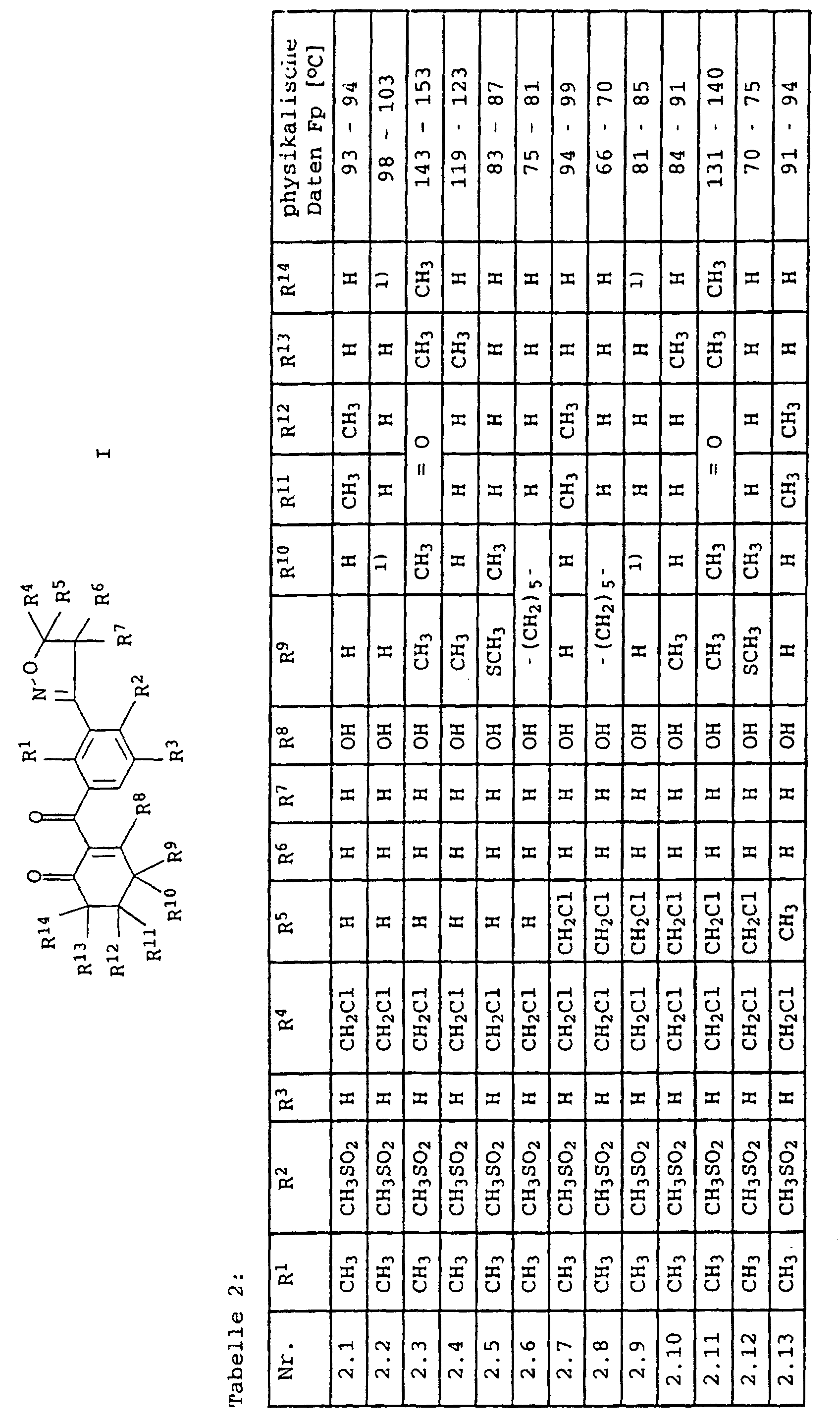

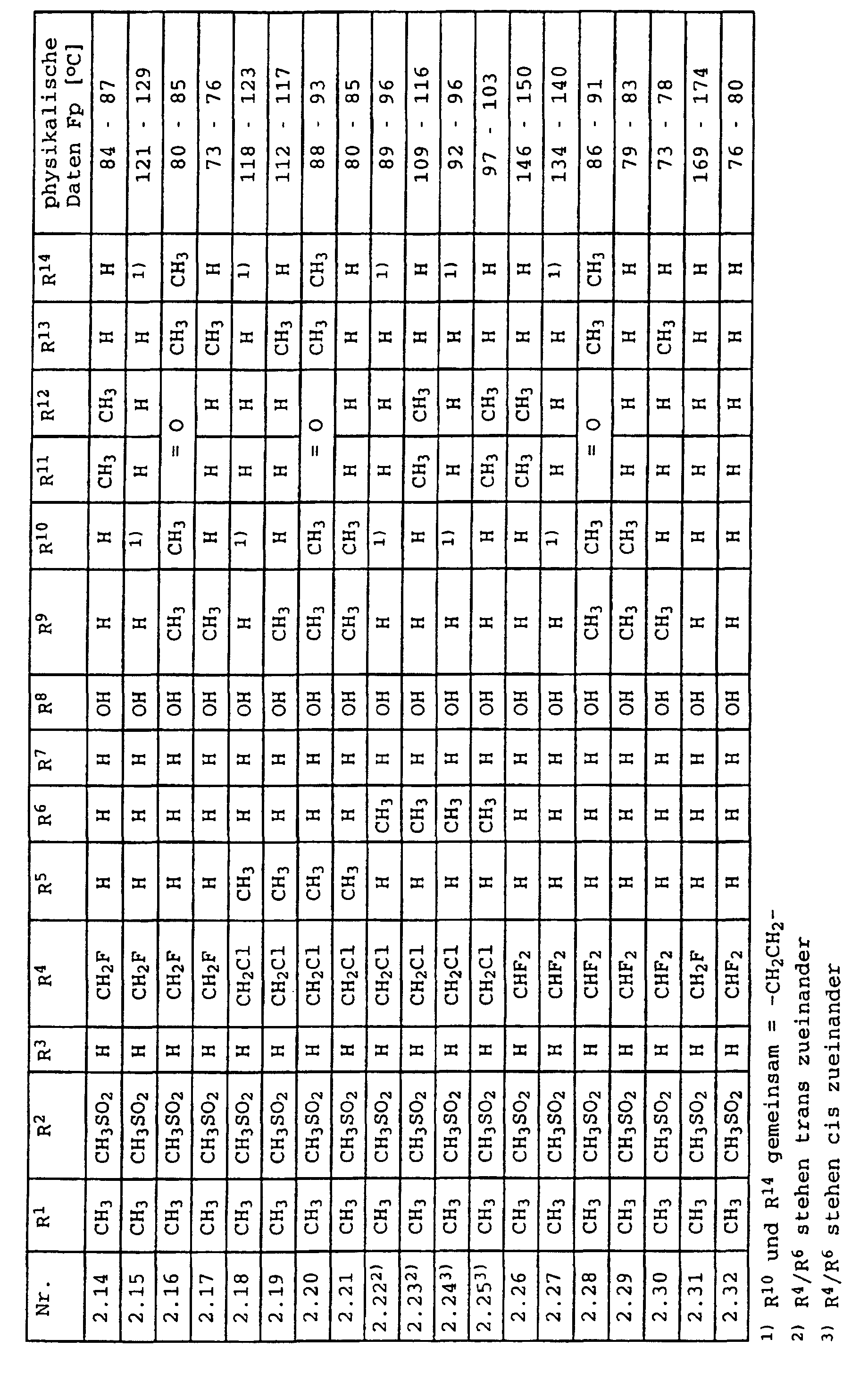

Die Halogenalkyl-substituierten 3-(4,5-Dihydroisoxazol-3-yl)-benzoylcyclohexenone der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen, wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wässrigen Lösungen, Pulvern, Suspensionen, auch hochprozentigen wässrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Hilfsstoffe kommen im Wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z. B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z. B. Amine wie N-Methylpyrrolidon und Wasser.

Wässrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Halogenalkyl-substituierten 3-(4,5-Dihydroisoxazol-3-yl)-benzoylcyclohexenone als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z. B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löss, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im Allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 95 % bis 100 % (nach NMR-Spektrum) eingesetzt.

Die folgenden Formulierungsbeispiele verdeutlichen die Herstellung solcher Zubereitungen:
I. 20 Gewichtsteile der Verbindung Nr. 2.1 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100 000 Gewichtsteilen wasser erhält man eine wässrige Dispersion, die 0,02 Gew.-% des Wirkstoffs enthält.
II. 20 Gewichtsteile der Verbindung Nr. 2.1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100 000 Gewichtsteilen Wasser erhält man eine wässrige Dispersion, die 0,02 Gew.-% des Wirkstoffs enthält.
III. 20 Gewichtsteile des Wirkstoffs Nr. 2.1 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wässrige Dispersion, die 0,02 Gew.-% des Wirkstoffs enthält.
IV. 20 Gewichtsteile des Wirkstoffs Nr. 2.1 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.
V. 3 Gewichtsteile des Wirkstoffs Nr. 2.1 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 2.1 werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil des Wirkstoffs Nr. 2.1 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil des Wirkstoffs Nr. 2.1 wird in einer Mischung gelöst, die aus 80 Gewichtsteilen Cyclohexanon und 20 Gewichtsteilen Wettol® EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, dass die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die Halogenalkyl-substituierte 3-(4,5-Dihydroisoxazol-3-yl)-benzoylcyclohexenone der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3- cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der Halogenalkyl-substituierte 3-(4,5-Dihydroisoxazol-3-yl)-benzoylcyclohexenone der Formel I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,25 bzw. 0,125 kg/ha a.S. (aktive Substanz).

Die Pflanzen wurden artenspezifisch bei Temperaturen von 10 bis 25°C bzw. 20 bis 35 °C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Abutilon theophrasti | Chinesischer Hanf | velvet leaf |
| Avena fatua | Flughafer | wild oat |
| Brachiaria plantaginea | | alexandergrass |
| Chenopodium album | Weißer Gänsefuß | lambsquaters |
| Polygonum persicaria | Flohknöterich | ladysthumb |
| Setaria faberi | Große Borstenhirse | giant foxtail |

Die Aufwandmengen von 0,25 bzw. 0,125 kg/ha zeigte die Verbindung 2.1 (Tabelle 2) im Nachauflauf eine sehr gute Wirkung gegen die oben genannten unerwünschten Pflanzen.

## Patentansprüche

1. Halogenalkyl-substituierte 3-(4,5-Dihydroisoxazol-3-yl)-Benzoylcyclohexenone der Formel I 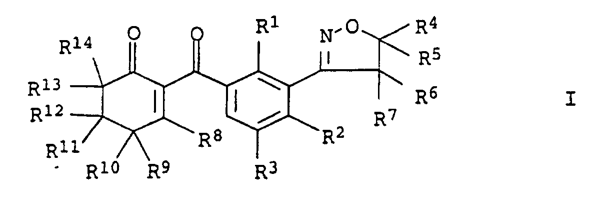 worin
R¹ für C₁-C₆-Alkyl, C₁-C₆-Halogenalkyl, C₁-C₆-Alkoxy oder C₁-C₆-Halogenalkoxy;
R² für C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl, Halogen, Cyano oder Nitro;
R³ für Wasserstoff, C₁-C₆-Alkyl oder Halogen;
R⁴ für C₁-C₄-Halogenalkyl steht;
R⁵, R⁶, R⁷ unabhängig voneinander für Wasserstoff, C₁-C₄-Alkyl oder C₁-C₄-Halogenalkyl stehen;
R⁸ für Hydroxy, Mercapto, Halogen, OR¹⁵, SR¹⁵, SOR¹⁶ oder SO₂R¹⁶ steht;
R⁹, R¹³ unabhängig voneinander für Wasserstoff, C₁-C₄-Alkyl, C₁-C₄-Alkylthio oder C₁-C₄-Alkoxycarbonyl stehen;
R¹⁰, R¹², R¹⁴ unabhängig voneinander für Wasserstoff oder C₁-C₄-Alkyl stehen;
R¹¹ für Wasserstoff, Hydroxy, Halogen, C₁-C₆-Alkyl, C₁-C₆-Halogenalkyl, Di(C₁-C₆-alkoxy)methyl, (C₁-C₆-Alkoxy) (C₁-C₆-alkylthio)methyl, Di(C₁-C₆-alkylthio)methyl, C₁-C₆-Alkoxy, C₁-C₆-Halogenalkoxy, C₁-C₆-Alkylthio, C₁-C₆-Halogenalkylthio, C₁-C₆-Alkoxycarbonyl, C₁-C₆-Halogenalkoxycarbonyl,
1,3-Dioxolan-2-yl, 1,3-Dioxan-2-yl, 1,3-Oxathiolan-2-yl, 1,3-Oxathian-2-yl, 1,3-Dithiolan-2-yl oder 1,3-Dithian-2-yl steht, wobei die sechs letztgenannten Reste einen, zwei oder drei unter C₁-C₄-Alkyl ausgewählte Substituenten tragen könnnen;
oder
R⁹ und R¹⁰ oder R¹³ und R¹⁴ gemeinsam für C₁-C₅-Alkandiyl stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
R¹⁰ und R¹¹ oder R¹¹ und R¹⁴ gemeinsam für eine chemische Bindung oder C₁-C₅-Alkandiyl stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
oder
R¹⁰ und R¹⁴ gemeinsam für C₁-C₄-Alkandiyl stehen, das einen, zwei oder drei unter Halogen, Cyano, C₁-C₄-Alkyl, C₁-C₄-Alkoxycarbonyl ausgewählte Substituenten tragen kann;
oder
R¹¹ und R¹² gemeinsam für -O-(CH₂)ₚ-O-, -O-(CH₂)ₚ-S-, -S-(CH₂)ₚ-S-, -O-(CH₂)_{q}- oder -S-(CH₂)_{q}- stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
oder
R¹¹ und R¹² gemeinsam für ein Sauerstoffatom stehen;
R¹⁵ für C₁-C₆-Alkyl, C₃-C₆-Alkenyl, C₃-C₆-Halogenalkenyl, C₃-C₆-Alkinyl, C₃-C₆-Cycloalkyl, C₁-C₂₀-Alkylcarbonyl, C₂-C₂₀-Alkenylcarbonyl, C₂-C₆-Alkinylcarbonyl, C₁-C₆-Alkoxycarbonyl, C₃-C₆-Alkenyloxycarbonyl, C₃-C₆-Alkinyloxycarbonyl, C₁-C₆-Alkylthiocarbonyl, C₁-C₆-Alkylaminocarbonyl, C₃-C₆-Alkenylaminocarbonyl, C₃-C₆-Alkinylaminocarbonyl, N,N-Di(C₁-C₆-alkyl)aminocarbonyl, N-(C₃-C₆-Alkenyl)-N-(C₁-C₆-alkyl)aminocarbonyl, N-(C₃-C₆-Alkinyl)-N-(C₁-C₆-alkyl)aminocarbonyl, N-(C₁-C₆-Alkoxy)-N-(C₁-C₆-alkyl)aminocarbonyl, N,N-Di(C₁-C₆-alkyl)aminothiocarbonyl, C₁-C₆-Alkoxyimino-C₁-C₆-alkyl steht, wobei die genannten Alkyl-, Alkoxy- und Cycloalkylreste partiell oder vollständig halogeniert sein können und/oder einen, zwei oder drei unter Cyano, C₁-C₄-Alkoxy, C₁-C₄-Alkylthio, Di(C₁-C₄-alkyl)amino, C₁-C₄-Alkylcarbonyl, C₁-C₄-Alkoxycarbonyl, C₁-C₄-Alkoxy-C₁-C₄-alkoxycarbonyl, C₁-C₄-Alkylaminocarbonyl, N,N-Di-(C₁-C₄-alkyl)aminocarbonyl oder C₃-C₆-Cycloalkyl ausgewählte Substituenten tragen können; Phenyl, Phenyl-C₁-C₆-alkyl, Phenylcarbonyl-C₁-C₆-alkyl, Phenylcarbonyl, Phenoxycarbonyl, Phenoxythiocarbonyl, Heterocycyl, Heterocyclyl-C₁-C₆-alkyl, Heterocyclylcarbonyl-C₁-C₆-alkyl, Heterocyclylcarbonyl, Heterocyclyloxycarbonyl, Heterocyclyloxythiocarbonyl, wobei der Phenyl- oder Heterocyclylrest der genannten Reste partiell oder vollständig halogeniert sein kann und/oder einen, zwei oder drei unter Nitro, Cyano, C₁-C₄-Alkyl, C₁-C₄-Halogenalkyl, C₁-C₄-Alkoxy oder C₁-C₄-Halogenalkoxy ausgewählte Substituenten tragen kann;
R¹⁶ für C₁-C₆-Alkyl, C₃-C₆-Alkenyl, C₃-C₆-Halogenalkenyl, C₃-C₆-Alkinyl oder C₃-C₆-Cycloalkyl steht, wobei die genannten Alkyl- und Cycloalkylreste partiell oder vollständig halogeniert sein können und/oder einen, zwei oder drei unter Cyano, C₁-C₄-Alkoxy, C₁-C₄-Alkylthio, Di(C₁-C₄-alkyl)amino, C₁-C₄-Alkylcarbonyl, C₁-C₄-Alkoxycarbonyl, C₁-C₄-Alkoxy-C₁-C₄-alkoxycarbonyl, C₁-C₄-Alkylaminocarbonyl, N,N-Di-(C₁-C₄-alkyl)aminocarbonyl oder C₃-C₆-Cycloalkyl ausgewählte Substituenten tragen können;
Phenyl, Phenyl-C₁-C₆-alkyl, Phenylcarbonyl-C₁-C₆-alkyl, Heterocycyl, Heterocyclyl-C₁-C₆-alkyl oder Heterocyclylcarbonyl-C₁-C₆-alkyl, wobei der Phenyl- oder Heterocyclylrest der genannten Reste partiell oder vollständig halogeniert sein kann und/oder einen, zwei oder drei unter Nitro, Cyano, C₁-C₄-Alkyl, C₁-C₄-Halogenalkyl, C₁-C₄-Alkoxy oder C₁-C₄-Halogenalkoxy ausgewählte Substituenten tragen kann;
p für 2, 3 oder 4;
q für 1, 2, 3, 4 oder 5 steht;
sowie deren landwirtschaftlich brauchbaren Salze.

2. Halogenalkyl-substituierte 3-(4,5-Dihydroisoxazol-3-yl)-Benzoylcyclohexenone nach Anspruch 1, worin
R⁴ für Fluormethyl, Chlormethyl, Brommethyl, Trifluormethyl, 1-Chlor-1-ethyl, 1-Fluor-1-ethyl oder Pentafluorethyl steht.

3. Halogenalkyl-substituierte 3-(4,5-Dihydroisoxazol-3-yl)-Benzoylcyclohexenone nach Anspruch 1 oder 2, worin
R¹ für C₁-C₄-Alkyl;
R² für C₁-C₆-Halogenalkyl, C₁-C₆-Alkylsulfonyl, Halogen oder Nitro; und
R³ für Wasserstoff steht.

4. Halogenalkyl-substituierte 3-(4,5-Dihydroisoxazol-3-yl)-Benzoylcyclohexenone nach Anspruch 1, 2 oder 3, worin
R⁹ und R¹³ unabhängig voneinander für Wasserstoff, C₁-C₄-Alkyl oder C₁-C₄-Alkylthio;
R¹⁰, R¹², R¹⁴ unabhängig voneinander für Wasserstoff oder Methyl stehen und
R¹¹ für Wasserstoff, Hydroxy, C₁-C₆-Alkyl oder Di(C₁-C₆-alkoxy)methyl steht;
oder
R⁹ und R¹⁰ oder R¹⁰ und R¹¹ oder R¹¹ und R¹⁴ oder R¹⁰ und R¹⁴ oder R¹³ und R¹⁴ gemeinsam für C₁-C₅-Alkandiyl stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
oder
R¹¹ und R¹² gemeinsam für ein Sauerstoffatom stehen.

5. Halogenalkyl-substituierte 3-(4,5-Dihydroisoxazol-3-yl)-Benzoylcyclohexenone nach einem der vorhergehenden Ansprüche, wobei
R⁸ für Hydroxy, OR¹⁵ oder SR¹⁵; und
R¹⁵ für C₁-C₆-Alkyl, C₁-C₂₀-Alkylcarbonyl, C₁-C₆-Alkoxycarbonyl, C₁-C₆-Alkylthiocarbonyl, wobei die Alkyl- und Alkoxyreste partiell oder vollständig halogeniert sein können und/oder einen, zwei oder drei unter Cyano, C₁-C₄-Alkoxy, C₁-C₄-Alkylthio, C₁-C₄-Alkylcarbonyl, C₁-C₄-Alkoxycarbonyl oder C₃-C₆-Cycloalkyl ausgewählte Substituenten tragen können;
Phenyl, Phenyl-C₁-C₄-alkyl, Phenylcarbonyl-C₁-C₄-alkyl, Phenylcarbonyl, Phenoxycarbonyl, Heterocyclyl, Heterocyclyl-C₁-C₄-alkyl, Heterocyclylcarbonyl-C₁-C₄-alkyl, Heterocyclyloxycarbonyl steht, wobei der Phenyl- oder Heterocyclylrest der genannten Reste partiell oder vollständig halogeniert sein kann und/oder einen, zwei oder drei unter Nitro, Cyano, C₁-C₄-Alkyl, C₁-C₄-Halogenalkyl, C₁-C₄-Alkoxy oder C₁-C₄-Halogenalkoxy ausgewählten Substituenten tragen kann.

6. Verfahren zur Herstellung von Halogenalkyl-substituierten 3-(4,5-Dihydroisoxazol-3-yl)-Benzoylcyclohexenonen der Formel I mit R⁸ = OH nach Anspruch 1, bei dem man ein Cyclohexenon der Formel II 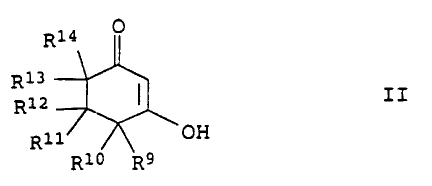 mit einem Benzoesäurederivat III 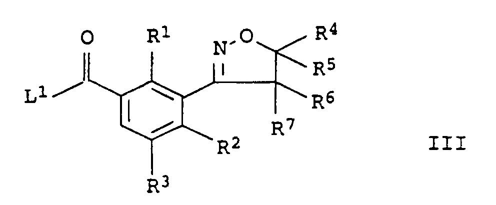 worin R¹ bis R⁷ und R⁹ bis R¹⁴ die in Anspruch 1 angegebene Bedeutung haben und L¹ für Hydroxy oder eine nucleophil verdrängbare Abgangsgruppe steht, acyliert und das Acylierungsprodukt zur einer Verbindung der Formel I, worin R⁸ für Hydroxy steht, umlagert.

7. Verfahren zur Herstellung von Verbindungen der Formel I mit R⁸ = OR¹⁵ oder SR¹⁵ nach Anspruch 1, bei dem man eine Verbindung der Formel I mit R⁸ = OH oder SH, 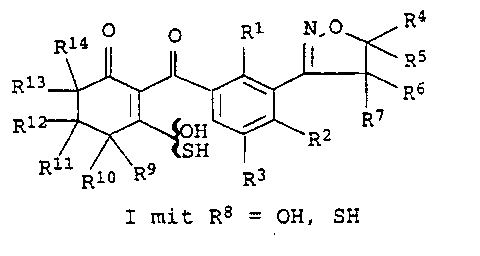 worin R¹ bis R⁷ und R⁹ bis R¹⁴ die in Anspruch 1 angegebene Bedeutung haben, mit einer Verbindung der Formel VIII
L⁴-R¹⁵ VIII
wobei die Variable R¹⁵ die in Anspruch 1 angegebene Bedeutung hat und L⁴ für eine nucleophil verdrängbare Abgangsgruppe steht, umsetzt.

8. Verfahren zur Herstellung von Verbindungen der Formel I mit R⁸ = Halogen nach Anspruch 1, bei dem man eine Verbindung der Formel I mit R⁸ = OH, 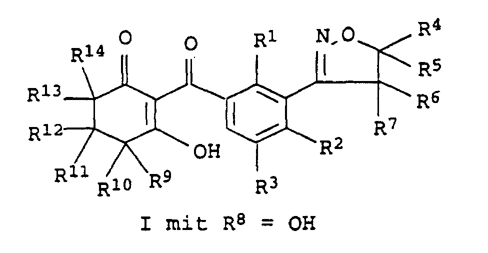 wobei die Variablen R¹ bis R⁷ und R⁹ bis R¹⁴ die in Anspruch 1 genannte Bedeutung haben, mit einem Halogenierungsmittel umsetzt.

9. Verfahren zur Herstellung von Verbindungen der Formel I mit R⁸ = Mercapto, OR¹⁵ oder SR¹⁵ nach Anspruch 1, bei dem man eine Verbindung der Formel I mit R⁸ = Halogen, 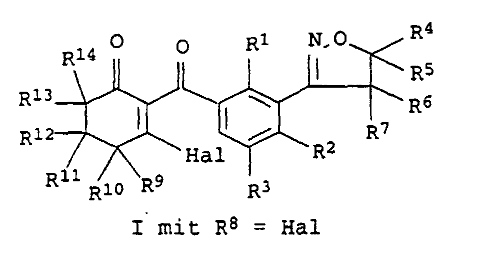 worin die Variablen R¹ bis R⁷ und R⁹ bis R¹⁴ die in Anspruch 1 angegebene Bedeutung haben, mit einer Verbindung der Formel IX
H₂S oder HOR¹⁵ oder HSR¹⁵ IX
wobei R¹⁵ die in Anspruch 1 angegebene Bedeutung hat, gegebenenfalls in Gegenwart einer Base umsetzt.

10. Verfahren zur Herstellung von Verbindungen der Formel I mit R⁸ = SOR¹⁶ oder SO₂R¹⁶ nach Anspruch 1, bei dem man eine Verbindung der Formel I mit R⁸ = SR¹⁶, 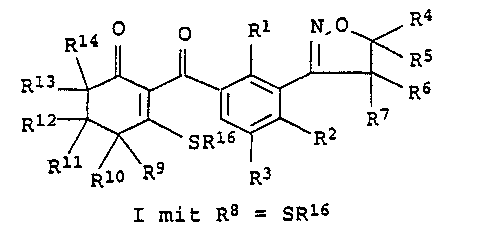 wobei die Variablen R¹ bis R⁷ und R⁹ bis R¹⁴ die in Anspruch 1 genannte Bedeutung haben, mit einem Oxidationsmittel umsetzt.

11. Mittel, enthaltend eine herbizid wirksame Menge mindestens eines Halogenalkyl-substituierten 3-(4,5-Dihydroisoxazol-3-yl) -Benzoylcyclohexenons der Formel I oder eines landwirtschaftlich brauchbaren Salzes davon gemäß den Ansprüchen 1 bis 5, und für die Formulierung von Pflanzenschutzmitteln übliche Hilfsmittel.

12. Verfahren zur Bekämpfung von unerwünschtem Pflanzenwuchs, **dadurch gekennzeichnet, dass** man eine herbizid wirksame Menge mindestens eines Halogenalkyl-substituierten 3-(4,5-Dihydroisoxazol-3-yl)-Benzoylcyclohexenons der Formel I oder eines landwirtschaftlich brauchbaren Salzes davon I gemäß den Ansprüchen 1 bis 5, auf Pflanzen, deren Lebensraum und/oder auf Samen einwirken lässt.

13. Verwendung der Halogenalkyl-substituierten 3-(4,5-Dihydroisoxazol-3-yl)-Benzoylcyclohexenone der Formel I und/oder deren landwirtschaftlich brauchbaren Salze gemäß den Ansprüchen 1 bis 5 als Herbizide.

## Claims

1. A haloalkyl-substituted 3-(4,5-dihydroisoxazol-3-yl)benzoylcyclohexenone of the formula I 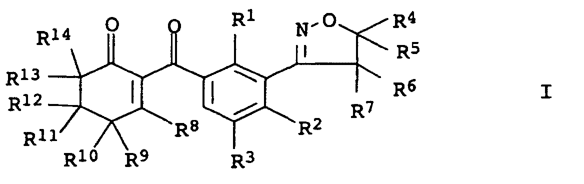 in which
R¹ is C₁-C₆-alkyl, C₁-C₆-haloalkyl, C₁-C₆-alkoxy or C₁-C₆-haloalkoxy;
R² is C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, halogen, cyano or nitro;
R³ is hydrogen, C₁-C₆-alkyl or halogen;
R⁴ is C₁-C₄-haloalkyl;
R⁵, R⁶, R⁷ independently of one another are hydrogen, C₁-C₄-alkyl or C₁-C₄-haloalkyl;
R⁸ is hydroxyl, mercapto, halogen, OR¹⁵, SR¹⁵, SOR¹⁶ or SO₂R¹⁶;
R⁹, R¹³ independently of one another are hydrogen, C₁-C₄-alkyl, C₁-C₄-alkylthio or C₁-C₄-alkoxycarbonyl;
R¹⁰, R¹², R¹⁴ independently of one another are hydrogen or C₁-C₄-alkyl;
R¹¹ is hydrogen, hydroxyl, halogen, C₁-C₆-alkyl, C₁-C₆-haloalkyl, di(C₁-C₆-alkoxy)methyl, (C₁-C₆-alkoxy)(C₁-C₆-alkylthio)methyl, di(C₁-C₆-alkylthio)methyl, C₁-C₆-alkoxy, C₁-C₆-haloalkoxy, C₁-C₆-alkylthio, C₁-C₆-haloalkylthio, C₁-C₆-alkoxycarbonyl, C₁-C₆-haloalkoxycarbonyl,
1,3-dioxolan-2-yl, 1,3-dioxan-2-yl, 1,3-oxathiolan-2-yl, 1,3-oxathian-2-yl, 1,3-dithiolan-2-yl or 1,3-dithian-2-yl, where the six lastmentioned radicals may carry one, two or three substituents selected from C₁-C₄-alkyl;
or
R⁹ and R¹⁰ or R¹³ and R¹⁴ together are C₁-C₅-alkanediyl which may carry one, two or three substituents selected from the group consisting of halogen, cyano, C₁-C₄-alkyl, C₁-C₄-haloalkyl and C₁-C₄-alkoxycarbonyl;
or
R¹⁰ and R¹¹ or R¹¹ and R¹⁴ together are a chemical bond or C₁-C₅-alkanediyl which may carry one, two or three substituents selected from the group consisting of halogen, cyano, C₁-C₄-alkyl, C₁-C₄-haloalkyl and C₁-C₄-alkoxycarbonyl;
or
R¹⁰ and R¹⁴ together are C₁-C₄-alkanediyl which may carry one, two or three substituents selected from the group consisting of halogen, cyano, C₁-C₄-alkyl, C₁-C₄-alkoxycarbonyl;
or
R¹¹ and R¹² together are -O-(CH₂)ₚ-O-, -O-(CH₂)ₚ-S-, -S-(CH₂)ₚ-S-, -O-(CH₂)_{q}- or -S-(CH₂)_{q}- which may carry one, two or three substituents selected from the group consisting of halogen, cyano, C₁-C₄-alkyl, C₁-C₄-haloalkyl and C₁-C₄-alkoxycarbonyl;
or
R¹¹ and R¹² together are an oxygen atom;
R¹⁵ is C₁-C₆-alkyl, C₃-C₆-alkenyl, C₃-C₆-haloalkenyl, C₃-C₆-alkynyl, C₃-C₆-cycloalkyl, C₁-C₂₀-alkylcarbonyl, C₂-C₂₀-alkenylcarbonyl, C₂-C₆-alkynylcarbonyl, C₁-C₆-alkoxycarbonyl, C₃-C₆-alkenyloxycarbonyl, C₃-C₆-alkynyloxycarbonyl, C₁-C₆-alkylthiocarbonyl, C₁-C₆-alkylaminocarbonyl, C₃-C₆-alkenylaminocarbonyl, C₃-C₆-alkynylaminocarbonyl, N,N-di(C₁-C₆-alkyl)aminocarbonyl, N-(C₃-C₆-alkenyl)-N-(C₁-C₆-alkyl)aminocarbonyl, N-(C₃-C₆-alkynyl)-N-(C₁-C₆-alkyl)aminocarbonyl, N-(C₁-C₆-alkoxy)-N-(C₁-C₆-alkyl)aminocarbonyl, N,N-di(C₁-C₆-alkyl)aminothiocarbonyl, C₁-C₆-alkoxyimino-C₁-C₆-alkyl, where the alkyl, alkoxy and cycloalkyl radicals mentioned may be partially or fully halogenated and/or may carry one, two or three substituents selected from the group consisting of cyano, C₁-C₄-alkoxy, C₁-C₄-alkylthio, di(C₁-C₄-alkyl)amino, C₁-C₄-alkylcarbonyl, C₁-C₄-alkoxycarbonyl, C₁-C₄-alkoxy-C₁-C₄-alkoxycarbonyl, C₁-C₄-alkylaminocarbonyl, N,N-di(C₁-C₄-alkyl)aminocarbonyl and C₃-C₆-cycloalkyl;
is phenyl, phenyl-C₁-C₆-alkyl, phenylcarbonyl-C₁-C₆-alkyl, phenylcarbonyl, phenoxycarbonyl, phenoxythiocarbonyl, heterocyclyl, heterocyclyl-C₁-C₆-alkyl, heterocyclylcarbonyl-C₁-C₆-alkyl, heterocyclylcarbonyl, heterocyclyloxycarbonyl, heterocyclyloxythiocarbonyl, where the phenyl or heterocyclyl radical of the radicals mentioned may be partially or fully halogenated and/or may carry one, two or three substituents selected from the group consisting of nitro, cyano, C₁-C₄-alkyl, C₁-C₄-haloalkyl, C₁-C₄-alkoxy and C₁-C₄-haloalkoxy;
R¹⁶ is C₁-C₆-alkyl, C₃-C₆-alkenyl, C₃-C₆-haloalkenyl, C₃-C₆-alkynyl or C₃-C₆-cycloalkyl, where the alkyl and cycloalkyl radicals mentioned may be partially or fully halogenated and/or may carry one, two or three substituents selected from the group consisting of cyano, C₁-C₄-alkoxy, C₁-C₄-alkylthio, di(C₁-C₄-alkyl)amino, C₁-C₄-alkylcarbonyl, C₁-C₄-alkoxycarbonyl, C₁-C₄-alkoxy-C₁-C₄-alkoxycarbonyl, C₁-C₄-alkylaminocarbonyl, N,N-di(C₁-C₄-alkyl)aminocarbonyl and C₃-C₆-cycloalkyl;
is phenyl, phenyl-C₁-C₆-alkyl, phenylcarbonyl-C₁-C₆-alkyl, heterocyclyl, heterocyclyl-C₁-C₆-alkyl or heterocyclylcarbonyl-C₁-C₆-alkyl, where the phenyl or heterocyclyl radical of the radicals mentioned may be partially or fully halogenated and/or may carry one, two or three substituents selected from the group consisting of nitro, cyano, C₁-C₄-alkyl, C₁-C₄-haloalkyl, C₁-C₄-alkoxy and C₁-C₄-haloalkoxy;
p is 2, 3 or 4;
q is 1, 2, 3, 4 or 5;
and its agriculturally useful salts.

2. A haloalkyl-substituted 3-(4,5-dihydroisoxazol-3-yl)benzoylcyclohexenone as claimed in claim 1 in which
R⁴ is fluoromethyl, chloromethyl, bromomethyl, trifluoromethyl, 1-chloro-1-ethyl, 1-fluoro-l-ethyl or pentafluoroethyl.

3. A haloalkyl-substituted 3-(4,5-dihydroisoxazol-3-yl)benzoylcyclohexenone as claimed in claim 1 or 2 in which
R¹ is C₁-C₄-alkyl;
R² is C₁-C₆-haloalkyl, C₁-C₆-alkylsulfonyl, halogen or nitro; and
R³ is hydrogen.

4. A haloalkyl-substituted 3-(4,5-dihydroisoxazol-3-yl)benzoylcyclohexenone as claimed in any of claims 1 to 3 in which
R⁹ and R¹³ independently of one another are hydrogen, C₁-C₄-alkyl or C₁-C₄-alkylthio;
R¹⁰, R¹², R¹⁴ independently of one another are hydrogen or methyl and
R¹¹ is hydrogen, hydroxyl, C₁-C₆-alkyl or di(C₁-C₆-alkoxy)methyl;
or
R⁹ and R¹⁰ or R¹⁰ and R¹¹ or R¹¹ and R¹⁴ or R¹⁰ and R¹⁴ or R¹³ and R¹⁴ together are C₁-C₅-alkanediyl which may carry one, two or three substituents selected from the group consisting of halogen, cyano, C₁-C₄-alkyl, C₁-C₄-haloalkyl and C₁-C₄-alkoxycarbonyl;
or
R¹¹ and R¹² together are an oxygen atom.

5. A haloalkyl-substituted 3-(4,5-dihydroisoxazol-3-yl)benzoylcyclohexenone as claimed in any of the preceding claims, in which
R⁸ is hydroxyl, OR¹⁵ or SR¹⁵; and
R¹⁵ is C₁-C₆-alkyl, C₁-C₂₀-alkylcarbonyl, C₁-C₆-alkoxycarbonyl, C₁-C₆-alkylthiocarbonyl, where the alkyl and alkoxy radicals may be partially or fully halogenated and/or may carry one, two or three substituents selected from the group consisting of cyano, C₁-C₄-alkoxy, C₁-C₄-alkylthio, C₁-C₄-alkylcarbonyl, C₁-C₄-alkoxycarbonyl and C₃-C₆-cycloalkyl;
is phenyl, phenyl-C₁-C₄-alkyl, phenylcarbonyl-C₁-C₄-alkyl, phenylcarbonyl, phenoxycarbonyl, heterocyclyl, heterocyclyl-C₁-C₄-alkyl, heterocyclyicarbonyl-C₁-C₄-alkyl, heterocyclyloxycarbonyl, where the phenyl or heterocyclyl radical of the radicals mentioned may be partially or fully halogenated and/or may carry one, two or three substituents from the group consisting of nitro, cyano, C₁-C₄-alkyl, C₁-C₄-haloalkyl, C₁-C₄-alkoxy and C₁-C₄-haloalkoxy.

6. A process for preparing haloalkyl-substituted 3-(4,5-dihydroisoxazol-3-yl)benzoylcyclohexenones of the formula I where R⁸ = OH as claimed in claim 1, which comprises acylating a cyclohexenone of the formula II 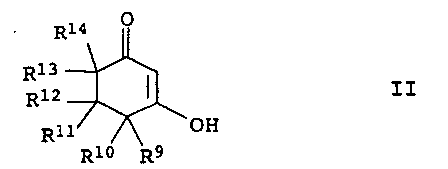 with a benzoic acid derivative III 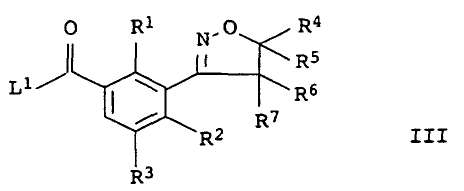 in which R¹ to R⁷ and R⁹ to R¹⁴ are as defined in claim 1 and L¹ is hydroxyl or a nucleophilically displaceable leaving group and rearranging the acylation product to a compound of the formula I in which R⁸ is hydroxyl.

7. A process for preparing compounds of the formula I where R⁸ = OR¹⁵ or SR¹⁵ as claimed in claim 1, which comprises reacting a compound of the formula I where R⁸ = OH or SH, 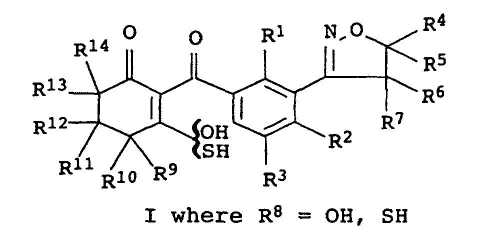 in which R¹ to R⁷ and R⁹ to R¹⁴ are as defined in claim 1 with a compound of the formula VIII
L⁴-R¹⁵ VIII
in which the variable R¹⁵ is as defined in claim 1 and L⁴ is a nucleophilically displaceable leaving group.

8. A process for preparing compounds of the formula I where R⁸ = halogen as claimed in claim 1, which comprises reacting a compound of the formula I where R⁸ = OH, 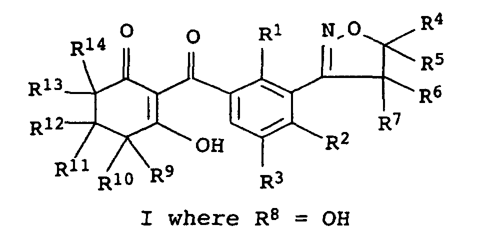 in which the variables R¹ to R⁷ and R⁹ to R¹⁴ are as defined in claim 1 with a halogenating agent.

9. A process for preparing compounds of the formula I where R⁸ = mercapto, OR¹⁵ or SR¹⁵ as claimed in claim 1, which comprises reacting a compound of the formula I where R⁸ = halogen, 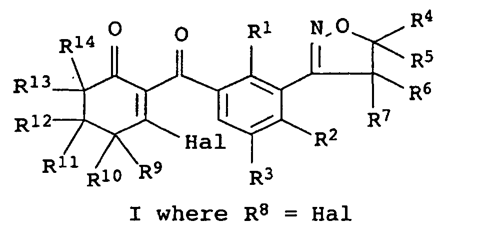 in which the variables R¹ to R⁷ and R⁹ to R¹⁴ are as defined in claim 1 with a compound of the formula IX
H₂S or HOR¹⁵ or HSR¹⁵ IX
in which R¹⁵ is as defined in claim 1, if appropriate in the presence of a base.

10. A process for preparing compounds of the formula I where R⁸ = SOR¹⁶ or SO₂R¹⁶ as claimed in claim 1, which comprises reacting a compound of the formula I where R⁸ = SR¹⁶, 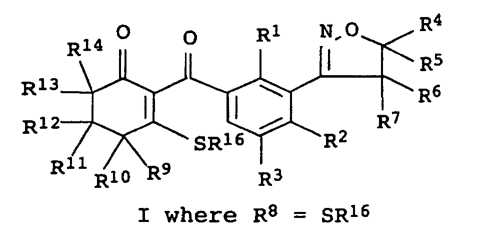 in which the variables R¹ to R⁷ and R⁹ to R¹⁴ are as defined in claim 1 with an oxidizing agent.

11. A composition, comprising a herbicidally effective amount of at least one haloalkyl-substituted 3-(4,5-dihydroisoxazol-3-yl)benzoylcyclohexenone of the formula I or an agriculturally useful salt thereof as claimed in any of claims 1 to 5 and auxiliaries customarily used for formulating crop protection agents.

12. A method for controlling undesirable vegetation, which comprises allowing a herbicidally effective amount of at least one haloalkyl-substituted 3-(4,5-dihydroisoxazol-3-yl)benzoylcyclohexenone of the formula I or an agriculturally useful salt thereof as claimed in any of claims 1 to 5, to act on plants, their habitat and/or on seeds.

13. The use of the haloalkyl-substituted 3-(4,5-dihydroisoxazol-3-yl)benzoylcyclohexenones of the formula I and/or of agriculturally useful salts thereof as claimed in any of claims 1 to 5 as herbicides.

## Revendications

1. 3-(4,5-dihydroisoxazol-3-yl)-benzoylcyclohexénones portant un ou plusieurs substituants halogénoalkyle, répondant à la formule (I) 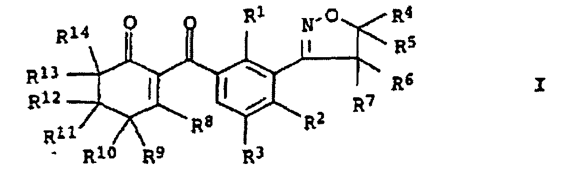 dans laquelle
R¹ représente un groupe alkyle en C₁-C₆, un groupe halogénoalkyle en C₁-C₆, un groupe alcoxy en C₁-C₆ ou un groupe halogénoalcoxy en C₁-C₆;
R² représente un groupe alkyle en C₁-C₆, un groupe halogénoalkyle en C₁-C₆, un groupe alcoxy en C₁-C₆, un groupe halogénoalcoxy en C₁-C₆, un groupe alkyl(en C₁-C₆)thio, un groupe halogénoalkyl(en C₁-C₆)thio, un groupe alkyl(en C₁-C₆)sulfinyle, un groupe halogénoalkyl(en C₁-C₆)sulfinyle, un groupe alkyl(en C₁-C₆)sulfonyle ou un groupe halogénoalkyl(en C₁-C₆)sulfonyle, un atome d'halogène, un groupe cyano ou un groupe nitro ;
R³ représente un atome d'hydrogène, un groupe alkyle en C₁-C₆ ou un atome d'halogène ;
R⁴ représente un groupe halogénoalkyle en C₁-C₄ ;
R⁵, R⁶, R⁷ représentent, indépendamment l'un de l'autre, un atome d'hydrogène, un groupe alkyle en C₁-C₄ ou un groupe halogénoalkyle en C₁-C₄;
R⁸ représente un groupe hydroxyle, un groupe mercapto, un atome d'halogène, un groupe OR¹⁵, un groupe SR¹⁵, un groupe SOR¹⁶ ou un groupe SO₂R¹⁶;
R⁹, R¹³ représentent, indépendamment l'un de l'autre, un atome d'hydrogène, un groupe alkyle en C₁-C₄, un groupe alkyl(en C₁-C₄)thio ou un groupe alcoxy(en C₁-C₄)carbonyle ;
R¹⁰, R¹², R¹⁴ représentent, indépendamment l'un de l'autre, un atome d'hydrogène ou un groupe alkyle en C₁-C₄ ;
R¹¹ représente un atome d'hydrogène, un groupe hydroxyle, un atome d'halogène, un groupe alkyle en C₁-C₆, un groupe halogénoalkyle en C₁-C₆, un groupe di(alcoxy en C₁-C₆)méthyle, un groupe (alcoxy en C₁-C₆)(alkyl(en C₁-C₆)-thio)méthyle, un groupe di(alkyl en C₁-C₆-thiométhyle, un groupe alcoxy en C₁-C₆, un groupe halogénoalcoxy en C₁-C₆, un groupe alkyl(en C₁-C₆)thio, un groupe halogénoalkyl(en C₁-C₆)thio, un groupe alcoxy(en C₁-C₆)carbonyle, un groupe halogénoalcoxy(en C₁-C₆)carbonyle ;
un groupe 1,3-dioxolan-2-yle, un groupe 1,3-dioxan-2-yle, un groupe 1,3-oxathiolan-2-yle, un groupe 1,3-oxathian-2-yle, un groupe 1,3-dithiolan-2-yle ou un groupe 1,3-dithian-2-yle, les six radicaux mentionnés en dernier lieu pouvant porter un, deux ou trois substituants choisis parmi des groupes alkyle en C₁-C₄ ;
ou
R⁹ et R¹⁰ ou R¹³ et R¹⁴ représentent ensemble un groupe alcane(en C₁-C₅)diyle qui peut porter un, deux ou trois substituants choisis parmi le groupe comprenant un atome d'halogène, un groupe cyano, un groupe alkyle en C₁-C₄, un groupe halogénoalkyle en C₁-C₄ ou un groupe alcoxy(en C₁-C₄)carbonyle ;
R¹⁰ et R¹¹ ou R¹¹ et R¹⁴ représentent ensemble une liaison chimique ou un groupe alcane(en C₁-C₅)diyle qui peut porter un, deux ou trois substituants choisis parmi le groupe comprenant un atome d'halogène, un groupe cyano, un groupe alkyle en C₁-C₄, un groupe halogénoalkyle en C₁-C₄ ou un groupe alcoxy(en C₁-C₄)carbonyle ;
ou
R¹⁰ et R¹⁴ représentent ensemble un groupe alcane(en C₁-C₄)diyle qui peut porter un, deux ou trois substituants choisis parmi le groupe comprenant un atome d'halogène, un groupe cyano, un groupe alkyle en C₁-C₄, un groupe alcoxy(en C₁-C₄)carbonyle ;
ou
R¹¹ et R¹² représentent ensemble un groupe -O-(CH₂)ₚ-O-, un groupe -O-(CH₂)ₚ-S-, un groupe -S-(CH₂)ₚ-S-, un groupe -O-(CH₂)_{q}- ou un groupe -S-(CH₂)_{q}-, qui peut porter un, deux ou trois substituants choisis parmi le groupe comprenant un atome d'halogène, un groupe cyano, un groupe alkyle en C₁-C₄, un groupe halogénoalkyle en C₁-C₄ ou un groupe alcoxy(en C₁-C₄)carbonyle ;
ou
R¹¹ et R¹² représentent ensemble un atome d'oxygène ;
R¹⁵ représente un groupe alkyle en C₁-C₆, un groupe alcényle en C₃-C₆, un groupe halogénoalcényle en C₃-C₆, un groupe alcynyle en C₃-C₆, un groupe cycloalkyle en C₃-C₆, un groupe alkyl(en C₁-C₂₀)carbonyle, un groupe alcényl(en C₂-C₂₀)carbonyle, un groupe alcynyl(en C₂-C₆)carbonyle, un groupe alcoxy(en C₁-C₆)carbonyle, un groupe alcényl(en C₃-C₆)oxycarbonyle, un groupe alcynyl(en C₃-C₆)oxycarbonyle, un groupe alkyl(en C₁-C₆)thiocarbonyle, un groupe alkyl(en C₁-C₆)aminocarbonyle, un groupe alcényl(en C₃-C₆)aminocarbonyle, un groupe alcynyl(en C₃-C₆)aminocarbonyle, un groupe N,N-di(alkyl en C₁-C₆)aminocarbonyle, un groupe N-(alcényl en C₃-C₆)-N-(alkyl en C₁-C₆)aminocarbonyle, un groupe N-(alcynyl en C₃-C₆)-N-(alkyl en C₁-C₆)aminocarbonyle, un groupe N-(alcoxy en C₁-C₆)-N-(alkyl en C₁-C₆)aminocarbonyle, un groupe N,N-di(alkyl en C₁-C₆)aminothiocarbonyle, un groupe alcoxy(en C₁-C₆)iminoalkyle en C₁-C₆, les radicaux alkyle, alcoxy et cycloalkyle mentionnés pouvant être partiellement ou complètement halogénés et/ou pouvant porter un, deux ou trois substituants choisis parmi le groupe comprenant un groupe cyano, un groupe alcoxy en C₁-C₄, un groupe alkyl(en C₁-C₄)thio, un groupe di(alkyl en C₁-C₄)amino, un groupe alkyl(en C₁-C₄)carbonyle, un groupe alcoxy(en C₁-C₄)carbonyle, un groupe alcoxy(en C₁-C₄)alcoxy(en C₁-C₄)carbonyle, un groupe alkyl(en C₁-C₄)aminocarbonyle, un groupe N,N-di(alkyl en C₁-C₄)aminocarbonyle ou un groupe cycloalkyle en C₃-C₆ ;
un groupe phényle, un groupe phénylalkyle en C₁-C₆, un groupe phénylcarbonylalkyle en C₁-C₆, un groupe phénylcarbonyle, un groupe phénoxycarbonyle, un groupe phénoxythiocarbonyle, un groupe hétérocyclyle, un groupe hétérocyclylalkyle en C₁-C₆, un groupe hétérocyclylcarbonylalkyle en C₁-C₆, un groupe hétérocyclylcarbonyle, un groupe hétérocyclyloxycarbonyle, un groupe hétérocyclyloxythiocarbonyle, le radical phényle ou le radical hétérocyclyle des radicaux mentionnés pouvant être partiellement ou complètement halogéné et/ou pouvant porter un, deux ou trois substituants choisis parmi le groupe comprenant un groupe nitro, un groupe cyano, un groupe alkyle en C₁-C₄, un groupe halogénoalkyle en C₁-C₄, un groupe alcoxy en C₁-C₄ ou un groupe halogénoalcoxy en C₁-C₄ ;
R¹⁶ représente un groupe alkyle en C₁-C₆, un groupe alcényle en C₃-C₆, un groupe halogénoalcényle en C₃-C₆, un groupe alcynyle en C₃-C₆ ou un groupe cycloalkyle en C₃-C₆, les radicaux alkyle et cycloalkyle mentionnés pouvant être partiellement ou complètement halogénés et/ou pouvant porter un, deux ou trois substituants choisis parmi le groupe comprenant un groupe cyano, un groupe alcoxy en C₁-C₄, un groupe alkyl(en C₁-C₄)thio, un groupe di(alkyl en C₁-C₄)amino, un groupe alkyl(en C₁-C₄)carbonyle, un groupe alcoxy(en C₁-C₄)carbonyle, un groupe alcoxy(en C₁-C₄)alcoxy(en C₁-C₄)carbonyle, un groupe alkyl(en C₁-C₄)aminocarbonyle, un groupe N,N-di(alkyl en C₁-C₄)aminocarbonyle
ou un groupe cycloalkyle en C₃-C₆ ;
un groupe phényle, un groupe phénylalkyle en C₁-C₆, un groupe phénylcarbonylalkyle en C₁-C₆, un groupe hétérocyclyle, un groupe hétérocyclylalkyle en C₁-C₆ ou un groupe hétérocyclylcarbonylalkyle en C₁-C₆, le radical phényle ou le radical hétérocyclyle des radicaux mentionnés pouvant être partiellement ou complètement halogéné et/ou pouvant porter un, deux ou trois substituants choisis parmi le groupe comprenant un groupe nitro, un groupe cyano, un groupe alkyle en C₁-C₄, un groupe halogénoalkyle en C₁-C₄, un groupe alcoxy en C₁-C₄ ou un groupe halogénoalcoxy en C₁-C₄ ;
p représente 2, 3 ou 4 ;
q représente 1, 2, 3, 4 ou 5 ;
ainsi que leurs sels utiles en agriculture.

2. 3-(4,5-dihydroisoxazol-3-yl)-benzoylcyclohexénones portant un ou plusieurs substituants halogénoalkyle selon la revendication 1, dans lesquelles
R⁴ représente un groupe fluorométhyle, un groupe chlorométhyle, un groupe bromométhyle, un groupe trifluorométhyle, un groupe 1-chloro-1-éthyle, un groupe 1-fluoro-1-éthyle ou un groupe pentafluoroéthyle.

3. 3-(4,5-dihydroisoxazol-3-yl)-benzoylcyclohexénones portant un ou plusieurs substituants halogénoalkyle selon la revendication 1 ou 2, dans lesquelles
R¹ représente un groupe alkyle en C₁-C₄ ;
R² représente un groupe halogénoalkyle en C₁-C₆, un groupe alkyl(en C₁-C₆)sulfonyle, un atome d'halogène ou un groupe nitro ;
R³ représente un atome d'hydrogène.

4. 3-(4,5-dihydroisoxazol-3-yl)-benzoylcyclohexénones portant un ou plusieurs substituants halogénoalkyle selon la revendication 1, 2 ou 3, dans lesquelles
R⁹ et R¹³ représentent, indépendamment l'un de l'autre, un atome d'hydrogène, un groupe alkyle en C₁-C₄ ou un groupe alkyl(en C₁-C₄)thio ;
R¹⁰, R¹², R¹⁴ représentent, indépendamment l'un de l'autre, un atome d'hydrogène ou un groupe méthyle ;
R¹¹ représente un atome d'hydrogène, un groupe hydroxyle, un groupe alkyle en C₁-C₆ ou un groupe di(alcoxy en C₁-C₆)méthyle ;
ou
R⁹ et R¹⁰ ou R¹⁰ et R¹¹ ou R¹¹ et R¹⁴ ou R¹⁰ et R¹⁴ ou R¹³ et R¹⁴ représentent ensemble un groupe alcane(en C₁-C₅)diyle qui peut porter un, deux ou trois substituants choisis parmi le groupe comprenant un atome d'halogène, un groupe cyano, un groupe alkyle en C₁-C₄, un groupe halogénoalkyle en C₁-C₄ ou un groupe alcoxy(en C₁-C₄)carbonyle ;
ou
R¹¹ et R¹² représentent ensemble un atome d'oxygène.

5. 3-(4,5-dihydroisoxazol-3-yl)-benzoylcyclohexénones portant un ou plusieurs substituants halogénoalkyle selon l'une quelconque des revendications précédentes, dans lesquelles
R⁸ représente un groupe hydroxyle, un groupe OR¹⁵ ou un groupe SR¹⁵ ;
R¹⁵ représente un groupe alkyle en C₁-C₆, un groupe alkyl(en C₁-C₂₀)carbonyle, un groupe alcoxy(en C₁-C₆)carbonyle, un groupe alkyl(en C₁-C₆)thiocarbonyle, les radicaux alkyle et alcoxy mentionnés pouvant être partiellement ou complètement halogénés et/ou pouvant porter un, deux ou trois substituants choisis parmi le groupe comprenant un groupe cyano, un groupe alcoxy en C₁-C₄, un groupe alkyl(en C₁-C₄)thio, un groupe alkyl(en C₁-C₄)carbonyle, un groupe alcoxy(en C₁-C₄)carbonyle ou un groupe cycloalkyle en C₃-C₆ ;
un groupe phényle, un groupe phénylalkyle en C₁-C₄, un groupe phénylcarbonylalkyle en C₁-C₄, un groupe phénylcarbonyle, un groupe phénoxycarbonyle, un groupe hétérocyclyle, un groupe hétérocyclylalkyle en C₁-C₄, un groupe hétérocyclylcarbonylalkyle en C₁-C₄, un groupe hétérocyclyloxycarbonyle, le radical phényle ou le radical hétérocyclyle des radicaux mentionnés pouvant être partiellement ou complètement halogéné et/ou pouvant porter un, deux ou trois substituants choisis parmi le groupe comprenant un groupe nitro, un groupe cyano, un groupe alkyle en C₁-C₄, un groupe halogénoalkyle en C₁-C₄, un groupe alcoxy en C₁-C₄ ou un groupe halogénoalcoxy en C₁-C₄.

6. Procédé pour la préparation de 3-(4,5-dihydroisoxazol-3-yl)-benzoylcyclohexénones portant un ou plusieurs substituants halogénoalkyle répondant à la formule 1 dans laquelle R⁸ représente un groupe OH selon la revendication 1, dans lequel on acyle une cyclohexénone répondant à la formule II. 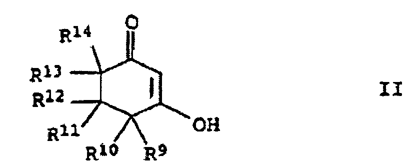 avec un dérivé III de l'acide benzoïque 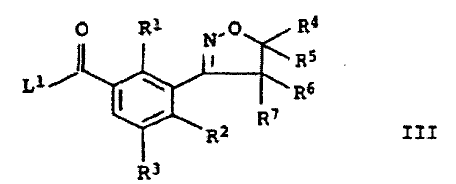 formules dans lesquelles R¹ à R⁷ et R⁹ à R¹⁴ ont la signification indiquée à la revendication 1 et L¹ représente un groupe hydroxyle ou un groupe de départ qui peut être soumis à un déplacement nucléophile et on transpose le produit d'acylation pour obtenir un composé répondant à la formule 1 dans laquelle R⁸ représente un groupe hydroxyle.

7. Procédé pour la préparation de composés répondant à la formule 1 dans laquelle R⁸ représente un groupe OR¹⁵ ou un groupe SR¹⁵ selon la revendication 1, dans lequel on fait réagir un composé répondant à la formule 1 dans laquelle R⁸ représente un groupe OH ou un groupe SH, 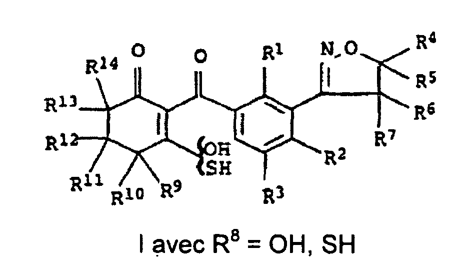 formule dans laquelle R¹ à R⁷ et R⁹ à R¹⁴ ont la signification indiquée à la revendication 1, avec un composé répondant à la formule VIII
L⁴-R¹⁵ VIII
la variable R¹⁵ possédant la signification indiquée à la revendication 1 et L⁴ représentant un groupe de départ apte à un déplacement nucléophile.

8. Procédé pour la préparation de composés répondant à la formule 1 dans laquelle R⁸ représente un atome d'halogène selon la revendication 1, dans lequel on fait réagir un composé répondant à la formule 1 dans laquelle R⁸ représente un groupe OH, 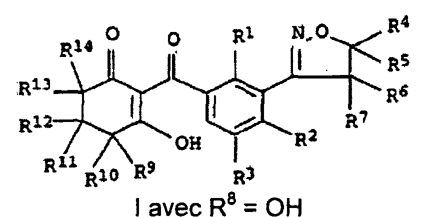 formule dans laquelle les variables R¹ à R⁷ et R⁹ à R¹⁴ ont la signification indiquée à la revendication 1, avec un agent d'halogénation.

9. Procédé pour la préparation de composés répondant à la formule I dans laquelle R⁸ représente un groupe mercapto, un groupe OR¹⁵ ou un groupe SR¹⁵ selon la revendication 1, dans lequel on fait réagir un composé répondant à la formule I dans laquelle R⁸ représente un atome d'halogène, 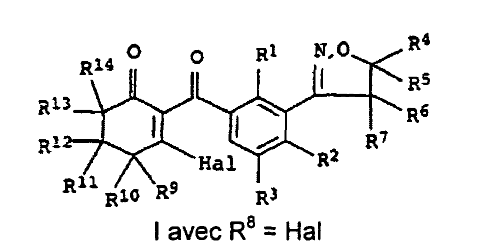 dans laquelle les variables R¹ à R⁷ et R⁹ à R¹⁴ ont la signification indiquée à la revendication 1, avec un composé répondant à la formule IX
H₂S ou HOR¹⁵ ou HSR¹⁵ IX
la variable R¹⁵ possédant la signification indiquée à la revendication 1, le cas échéant en présence d'une base.

10. Procédé pour la préparation de composés répondant à la formule I dans laquelle R⁸ représente un groupe SOR¹⁶ ou un groupe SO₂R¹⁶ selon la revendication 1, dans lequel on fait réagir un composé répondant à la formule 1 dans laquelle R⁸ représente un groupe SR¹⁶, 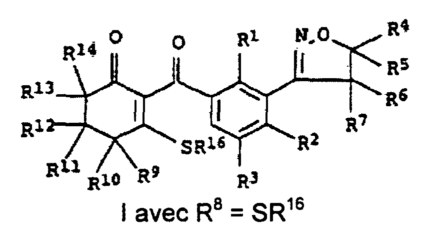 dans laquelle les variables R¹ à R⁷ et R⁹ à R¹⁴ ont la signification indiquée à la revendication 1, avec un un agent d'oxydation.

11. Agent contenant une quantité efficace du point de vue herbicide d'au moins une 3-(4,5-dihydroisoxazol-3-yl)-benzoyl-cyclohexénone portant un ou plusieurs substituants halogénoalkyle répondant à la formule I ou d'un de ses sels utiles en agriculture selon les revendications 1 à 5, et des adjuvants habituels pour la formulation d'agents de protection des plantes.

12. Procédé pour lutter contre la croissance non désirée de plantes, **caractérisé en ce qu'**on laisse agir une quantité efficace du point de vue herbicide d'au moins une 3-(4,5-dihydroisoxazol-3-yl)-benzoylcyclohexénone portant un ou plusieurs substituants halogénoalkyle répondant à la formule I ou d'un de ses sels utiles en agriculture selon les revendications 1 à 5, sur des plantes, sur leur biotope et/ou sur des semences.

13. Utilisation des 3-(4,5-dihydroisoxazol-3-yl)-benzoyl-cyclohexénones portant un ou plusieurs substituants halogénoalkyle répondant à la formule I et/ou de leurs sels utiles en agriculture selon les revendications 1 à 5, comme herbicides.
